# EUROPÄISCHE PATENTSCHRIFT

(11) **EP 2 386 203 B1**
(45) Veröffentlichungstag und Bekanntmachung des Hinweises auf die Patenterteilung: **20.11.2013**
(21) Anmeldenummer: 11175741.5
(22) Anmeldetag: 06.10.2009
(51) Int. Cl.: A01N 43/90, A01P 3/00

(54) **Verwendung von Dithiin-tetracarboximiden zum Bekämpfen phytopathogener Pilze**
Use of dithiin tetracarboximides for combating phytopathogenic fungi
Utilisation de dithine-tétracarboximidène pour combattre des champignons phytopathogènes

(30) Priorität: 15.10.2008 EP 08166621
(43) Veröffentlichungstag der Anmeldung: 16.11.2011
(62) Teilanmeldung aus: 09778847.5
(73) Patentinhaber: Bayer CropScience AG, 40789 Monheim am Rhein (DE)
(72) Erfinder: Seitz, Thomas, 40764 Langenfeld (DE); Wachendorff-Neumann, Ulrike, 56566 Neuwied (DE); Benting, Jürgen, 42799 Leichlingen (DE); Dahmen, Peter, 41470 Neuss (DE); Voerste, Arnd, 50674 Köln (DE); Dunkel, Ralf, 42799 Leichlingen (DE); Hillebrand, Stefan, 41462 Neuss (DE); Tietjen, Klaus-Günther, 40764 Langenfeld (DE); Brunet, Stéphane, 01390 Saint André de Corcy (FR)

(56) Entgegenhaltungen:
- ZENTZ ET AL: "Syntheses, in vitro antibacterial and antifungal activities of a series of N-alkyl, 1,4-dithiines", FARMACO, SOCIETA CHIMICA ITALIANA, PAVIA, IT, Bd. 60, Nr. 11-12, 1. November 2005 (2005-11-01), Seiten 944-947, XP005151567, ISSN: 0014-827X

## Beschreibung

Die vorliegende Erfindung betrifft die Verwendung von neuen und bekannten Dithiin-tetracarboximiden zum Schutz von geernteten Früchten oder Gemüse gegen phytopathogene Pilze.

Dithiin-tetracarboximide als solche sind bereits bekannt. Ebenso ist bekannt, dass diese Dithiin-tetracarboximide als Anthelminthika gegen innere Parasiten von Tieren, insbesondere Nematoden, verwendet werden können und insektizide Winkung aufweisen (vgl. US 3,364,229). Außerdem ist bekannt, das bestimmte Dithiin-tetracarboximide antibakterielle Wirkung besitzen und gegen menschliche Mykosen eine gewisse Wirkung aufweisen (vgl. II Farmaco, 2005, 60, 944-947). Weiterhin ist bekannt, dass Dithiin-tetracarboximide als Pigment in elektrophotographischen Photorezeptoren oder als Farbstoff in Lacken und Polymeren eingesetzt werden können (vgl. JP-A 10-251265, PL-B 143804).

Da sich die ökologischen und ökonomischen Anforderungen an moderne Fungizide laufend erhöhen, beispielsweise was Wirkungsspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Fungizide zu entwickeln, die zumindest in Teilbereichen die genannten Anforderungen besser erfüllen.

Es wurde nun gefunden, dass Dithiin-tetracarboximide der allgemeinen Formel (I) 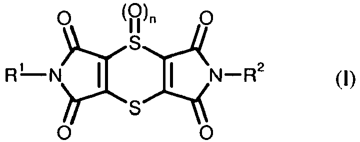 in welcher
- R¹ und R²: gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituier-tes Aryl oder Aryl-(C₁-C₄-alkyl) stehen,
- R³: für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
- R⁴: für Hydroxy, C₁-C₄-Alkyl oder C₁-C₄-Alkoxy steht,
- n: für 0 oder 1 steht,
sehr gut zum Bekämpfen phytopathogener Pilze verwendbar sind.

Erfindungsgemäße Dithiin-tetracarboximide der Formel (I) sowie gegebenenfalls deren Salze eignen sich sehr gut zur Bekämpfung pflanzenpathogener Schadpilze. Die vorgenannten erfindungsgemäßen Verbindungen zeigen vor allem eine fungizide Wirksamkeit und lassen sich sowohl im Pflanzenschutz, im Bereich Haushalt und Hygiene als auch im Materialschutz verwenden.

Die erfindungsgemäß verwendbaren Dithiin-tetracarboximide sind durch die Formel (I) allgemein definiert. Bevorzugt verwendbar sind Carboximide der Formel (I), in welcher die Reste die nachfolgenden Bedeutungen haben.
- R¹ und R²: sind bevorzugt gleich oder verschieden und stehen bevorzugt für Wasserstoff, für gegebenenfalls einfach oder mehrfach durch Fluor. Chlor. Brom, -OR³, -COR⁴ substituiertes C₁-C₆-Alkyl, gegebenenfalls einfach oder mehrfach durch Chlor. Methyl oder Trifluormethyl substituiertes C₃-C₇-Cycloalkyl, für jeweils gegebenenfalls einfach oder mehrfach durch Fluor, Chlor, Brom, Methyl, Trifluormethyl, -COR⁴, Sulfonylamino substituiertes Phenyl oder Phenyl-(C₁-C₄-alkyl).
- R¹ und R²: sind besonders bevorzugt gleich oder verschieden und stehen besonders bevorzugt für Wasserstoff, für gegebenenfalls einfach oder mehrfach durch Fluor, Chlor, Hydroxy, Methoxy, Ethoxy, Methylcarbonyloxy, Carboxyl substituiertes C₁-C₄-Alkyl, gegebenenfalls einfach oder mehrfach durch Chlor, Methyl oder Trifluormethyl substituiertes C₃-C₇-Cycloalkyl, für jeweils gegebenenfalls einfach bis dreifach durch Fluor, Chlor, Brom, Methyl, Trifluormethyl, -COR⁴, Sulfonylamino substituiertes Phenyl, Benzyl, 1-Phenethyl, 2-Phenethyl oder 2-Methyl-2-phenethyl.
- R¹ und R²: sind ganz besonders bevorzugt gleich oder verschieden und stehen ganz besonders bevorzugt für Wasserstoff, Methyl, Ethyl, n-Propyl, Isopropyl, 2,2-Difluorethyl, 2,2,2-Trifluorethyl, jeweils gegebenenfalls durch Chlor, Methyl oder Trifluormethyl substituiertes Cyclopropyl oder Cyclohexyl.
- R¹ und R²: stehen insbesondere bevorzugt gleichzeitig für Methyl.
- R³: steht bevorzugt für Wasserstoff, Methyl, Ethyl, Methylcarbonyl, Ethylcarbonyl oder für gegebenenfalls einfach oder mehrfach durch Fluor, Chlor, Methyl, Ethyl, n-Propyl, Isopropyl oder Trifluormethyl substituiertes Phenyl.
- R³: steht besonders bevorzugt für Wasserstoff, Methyl, Methylcarbonyl oder für Phenyl.
- R⁴: steht bevorzugt für Hydroxy, Methyl, Ethyl, Methoxy oder Ethoxy.
- R⁴: steht besonders bevorzugt für Hydroxy oder Methoxy.
- n: steht bevorzugt für 0.
- n: steht auch bevorzugt für 1.
- n: steht besonders bevorzugt für 0.

Im Einzelnen seien folgende Verbindungen genannt:
(1) 2,6-Dimethyl-1H,5H-[1,4]dithiino[2,3-c:5,6-c']dipyrrol-1,3,5,7(2H,6H)-tetron
(2) 2,6-Diethyl-1H,5H-[1,4]dithiino[2,3-c:5,6-c']dipyrrol-1,3,5,7(2H,6H)-tetron
(3) 2,6-Dipropyl-1H,5H-[1,4]dithiino[2,3-c:5,6-c']dipyrrol-1,3,5,7(2H,6H)-tetron
(4) 2,6-Di(propan-2-yl)-1H,5H-[1,4]dithiino[2,3-c:5,6-c']dipyrrol-1,3,5,7(2H,6H)-tetron
(5) 2,6-Dicyclopropyl-1H,5H-[1,4]dithiino[2,3-c:5,6-c']dipyrrol-1,3,5,7(2H,6H)-tetron
(6) 2,6-Bis(2,2,2-trifluorethyl)-1H,5H-[1,4]dithiino[2,3-c:5,6-c']dipyrrol-1,3,5,7(2H,6H)-tetron
(7) 2,6-Bis[1-(trifluormethyl)cyclopropyl]-1H,5H-[1,4]dithiino[2,3-c:5,6-c']dipyrrol-1,3,5,7(2H,6H)-tetron
(8) 1H,5H-[1,4]Dithiino[2,3-c:5,6-c']dipyrrol-1,3,5,7(2H,6H)-tetron
(9) 2,6-Bis(3,5-dichlorphenyl)-1H,5H-[1,4]dithiino[2,3-c:5.6-c']dipyrrol-1.3,5.7(2H,6H)-tetron
(10) 2.6-Diphenyl-1H,5H-[1.4]dithiino[2-3-c:5.6-c']dipyrrol-1.3,5.7(2H,6H)-tetron
(11) 2,6-Dibenzyl-1H,5H-[1,4]dithiino[2,3-c:5,6-c']dipyrrol-1.3,5.7(2H,6H)-tetron
(12) 2,6-Bis(2-methoxyethyl)-1H,5H-[1,4]dithiino[2,3-c:5,6-c']dipyrrol-1,3,5,7(2H,6H)-tetron
(13) 2,6-Bis(2-hydroxybutyl)-1H,5H-[1,4]dithiino[2,3-c:5,6-c']dipyrrol-1,3,5,7(2H,6H)-tetron
(14) 2,6-Bis(2-hydroxypropyl)-1H,5H-[1,4]dithiino[2,3-c:5,6-c']dipyrrol-1,3,5,7(2H,6H)-tetron
(15) 2,6-Bis(2-phenoxyethyl)-1H,5H-[1,4]dithiino[2,3-c:5,6-c']dipyrrol-1,3,5,7(2H,6H)-tetron
(16) 2,6-Bis(2-ethoxyethyl)-1H,5H-[1,4]dithiino[2,3-c:5,6-c']dipyrrol-1,3,5,7(2H,6H)-tetron
(17) 2,6-Bis(2-phenylpropan-2-yl)-1H,5H-[1,4]dithiino[2,3-c:5,6-c']dipyrrol-1,3,5,7(2H,6H)-tetron
(18) 2,6-Bis(1-phenylethyl)-1H,5H-[1,4]dithiino[2,3-c:5,6-c']dipyrrol-1,3,5,7(2H,6H)-tetron
(19) 2,6-Bis(2-methoxy-2-methylpropyl)-1H,5H-[1,4]dithiino[2,3-c:5,6-c']dipyrrol-1,3,5,7(2H,6H)-tetron
(20) 2,6-Di-tert-butyl-1H,5H-[1,4]dithiino[2,3-c:5.6-c']dipyrrol-1,3,5,7(2H,6H)-tetron
(21) (1,3,5,7-Tetraoxo-1,3,5,7-tetrahydro-2H,6H-[1,4]dithiino[2,3-c:5,6-c']dipyrrol-2,6-diyl)diethan-2,1-diyldiacetat
(22) 4,4'-(1,3,5,7-Tetraoxa-1,3,5,7-tetrahydro-2H,6H-[1,4]dithiino[2,3-c:5,6-c']dipyrrol-2,6-diyl)dibenzol-sulfonamid
(23) 2,2'-(1,3,5,7-Tetraoxo-1,3,5,7-tetrahydro-2H,6H-[1,4]dithiino[2,3-c:5,6-c']dipyrrol-2,6-diyl)diessigsäure
(24) 2,2'-(1,3,5,7-Tetraoxo-1,3,5,7-tetrahydro-2H,6H-[1,4]dithiino[2,3-c:5,6-c']dipyrrol-2,6-diyl)dipropansäure
(25) 2,2'-(1,3,5,7-Tetraoxo-1,3,5,7-tetrahydro-2H,6H-[1,4]dithiino[2,3-c:5,6-c']dipyrrol-2,6-diyl)dibutansäure
(26) 2,2'-(1,3,5,7-Tetraoxo-1,3,5,7-tetrahydro-2H,6H-[1,4]dithiino[2,3-c:5,6-c']dipyrrol-2,6-diyl)dihexansäure
(27) 2,2'-(1,3,5,7-Tetraoxo-1,3,5,7-tetrahydro-2H,6H-[1,4]dithiino[2,3-c:5,6-c']dipyrrol-2,6-diyl)bis(3,3-dimethylbutansäure)
(28) 3,3'-(1,3,5,7-Tetraoxo-1,3,5,7-tetrahydro-2H,6H-[1,4]dithiino[2,3-c:5,6-c']dipyrrol-2,6-diyl)dibutansäure
(29) 5,5'-(1,3,5,7-Tetraoxo-1,3,5,7-tetrahydro-2H,6H-[1,4]dithiino[2,3-c:5,6-c']dipyrrol-2,6-diyl)dipentansäure
(30) 2,6-Bis[3-(trifluormethyl)cyclohexyl]-1H,5H-[1,4]dithiino[2,3-c:5,6-c']dipyrrol-1,3,5,7(2H,6H)-tetron
(31) 2,6-Bis[3-(trifluormethyl)phenyl]-1H,5H-[1,4]dithiino[2,3-c:5,6-c']dipyrrol-1,3,5,7(2H,6H)-tetron
(32) 2,2'-(1,3,5,7-Tetraoxo-1,3,5,7-tetrahydro-2H,6H-[1,4]dithiino[2,3-c:5,6-c']dipyrrol-2,6-diyl)bis(3-phenyl-propansäure)
(33) 2,6-Bis(2-hydroxyethyl)-1H,5H-[1,4]dithiino[2,3-c:5,6-c']dipyrrol-1,3,5,7(2H,6H)-tetron
(34) 2,6-Bis(2-hydroxy-2-methylpropyl)-1H,5H-[1,4]dithiino[2,3-c:5,6-c']dipyrrol-1,3,5,7(2H,6H)-tetron
(35) (1,3,5,7-Tetraoxo-1,3,5,7-tetrahydro-2H,6H-[1,4]dithiino[2,3-c:5,6-c']dipyrrol)-2,6-diyl)dibutan-1,2-diyl-diacetat
(36) (1,3,5,7-Tetraoxo-1,3,5,7-tetrahydro-2H,6H-[1,4]dithiino[2,3-c:5,6-c']dipyrrol-2,6-diyl)dipropan-1,2-diyl-diacetat
(37) 2,6-Bis(hydroxymethyl)-1H,5H-[1,4]dithiino[2,3-c:5,6-c']dipyrrol-1,3,5,7(2H,6H)-tetron
(38) 2,6-Dimethyl-1H,5H-[1,4]dithiino[2,3-c:5,6-c']dipyrrol-1,3,5,7(2H,6H)-tetron-4-oxid
(39) 2-Ethyl-1H,5H-[1,4]dithiino[2,3-c:5,6-c']dipyrrol-1,3,5,7(2H,6H)-tetron
(40) Diethyl-2,2'-(1,3,5,7-tetraoxo-1,3,5,7-tetrahydro-2H,6H-[1,4]dithiino[2,3-c:5,6-c']dipyrrol-2,6-diyl)dihexanoat
(41) 2-[2-(1-Ethoxy-1-oxobutan-2-yl)-1,3,5,7-tetraoxo-2,3,5,7-tetrahydro-1H,6H-[1,4]dithiino[2,3-c:5,6-c']dipyrrol-6-yl]butansäure

Besonders bevorzugt verwendbar sind die Verbindungen (1), (2) und (3).

Neu sind Dithiin-tetracarboximide der Formel (I-a) 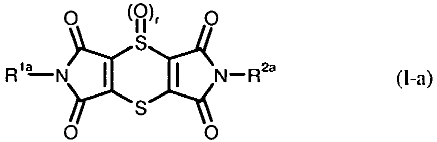 in welcher
- R^{1a} und R^{2a}: gleich oder verschieden sind und für einfach oder mehrfach durch Fluor, -OR^{3a}, -COR^{4a} substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, oder für einfach im Alkylteil durch -COR^{4a} substituiertes Aryl-(C₁-C₄-alkyl) stehen,
- R^{3a}: für C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
- R^{4a}: für Hydroxy, C₁-C₄-Alkyl oder C₁-C₄-Alkoxy steht,
- r: für 0 oder 1 steht,
- wobei R^{1a} und R^{2a}: nicht gleichzeitig für Acetoxymethyl oder Methoxymethyl stehen.
- R^{1a} und R^{2a}: sind bevorzugt gleich oder verschieden und stehen bevorzugt für einfach oder mehrfach durch Fluor, -OR^{3a}, -COR^{4a} substituiertes C₁-C₆-Alkyl, gegebenenfalls einfach oder mehrfach durch Chlor, Methyl oder Trifluormethyl substituiertes C₃-C₇-Cycloalkyl, oder für einfach im Alkylteil durch -COR^{4a} substituiertes Phenyl-(C₁-C₄-alkyl).
- R^{1a} und R^{2a}: sind besonders bevorzugt gleich oder verschieden und stehen besonders bevorzugt für einfach oder mehrfach durch Fluor, Hydroxy, Methoxy, Ethoxy, Methylcarbonyloxy, Carboxyl substituiertes C₁-C₄-Alkyl, gegebenenfalls einfach oder mehrfach durch Chlor, Methyl oder Trifluomiethyl substituiertes C₃-C₇-Cycloalkyl, oder für einfach im Alkylteil durch -COR^{4a} substituiertes 1-Phenethyl oder 2-Phenethyl.
- R^{1a} und R^{2a}: sind ganz besonders bevorzugt gleich oder verschieden und stehen ganz besonders bevorzugt für 2,2-Difluorethyl, 2,2,2-Trifluorethyl, jeweils gegebenenfalls durch Chlor, Methyl oder Trifluormethyl substituiertes Cyclopropyl oder Cyclohexyl.
- R^{3a}: steht bevorzugt für Methyl, Ethyl, Ethylcarbonyl, Ethylcarbonyl oder für gegebenenfalls einfach oder mehrfach durch Fluor, Chlor, Methyl, Ethyl, n-Propyl, Isopropyl oder Trifluormethyl substituiertes Phenyl.
- R^{3a}: steht besonders bevorzugt für Methyl, Methylcarbonyl oder für Phenyl.
- R^{4a}: steht bevorzugt für Hydroxy, Methyl, Ethyl, Methoxy oder Ethoxy.
- R^{4a}: steht besonders bevorzugt für Hydroxy oder Methoxy.
- r: steht bevorzugt für 0.
- r: steht auch bevorzugt für 1.
- r: steht besonders bevorzugt für 0.

Je nach Art der oben definierten Substituenten können die Verbindungen der Formel (I) saure oder basische Eigenschaften aufweisen und können mit anorganischen oder organischen Säuren oder mit Basen oder mit Metallionen Salze, gegebenenfalls auch innere Salze oder Addukte bilden.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt. Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden.

Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkylresten, Mono-, Di- und Trialkanolanüne von (C₁-C₄)-Alkanolen, Cholin sowie Chlorcholin.

Tragen die Verbindungen der Formel (I) Amino, Alkylamino oder andere, basische Eigenschaften induzierende Gruppen, so können diese Verbindungen mit Säuren zu Salzen umgesetzt werden.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄., und KHSO₄.

Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Die so erhältlichen Salze weisen ebenfalls fungizide Eigenschaften auf.

Die erfindungsgemäß verwendbaren Dithiin-tetracarboximide der Formel (I) können auf bekannte Weise hergestellt werden (vgl. US 3,364,229, Synthetic Commun. 2006, 36, 3591-3597 und II Farmaco 2005, 60, 944-947).

Beispielsweise wird in einem ersten Verfahren (vgl. II Farmaco 2005, 60, 944-947) in einer ersten Stufe Bernsteinsäureanhydrid der Formel (II) mit einem Amin der Formel (III) gegebenenfalls in Gegenwart eines Verdünndungsmittels umgesetzt. Anschließend werden dann die so erhaltenen Bernsteinsäuremonoamide der Formel (IV) mit einer Schwefelquelle (z.B. Thionylchlorid) umgesetzt. Je nach Reaktionsbedingungen können die Dithiin-diisoimide der Formel (V) isoliert werden, bevor man sie in die Dithiin-tetracarboximide der Formel (I-b) überführt. Die Herstellung der Dithiin-tetracarboximide der Formel (I) kann durch das folgende Schema veranschaulicht werden (R steht hierin für R¹ oder R²):

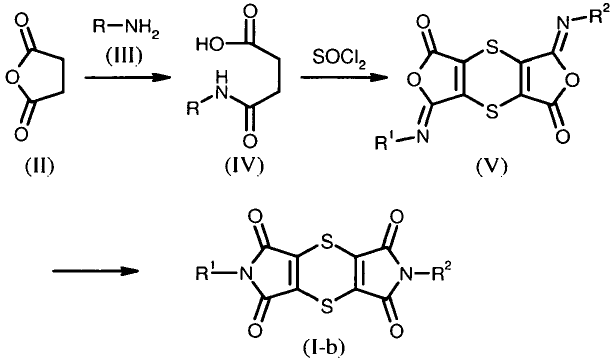

Auch die Dithiin-diisoimide der Formel (V) 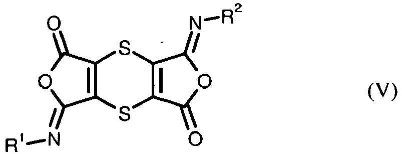 in welcher R¹ und R² die oben angegebenen Bedeutungen haben, eigenen sich zum Bekämpfen phytopathogener Pilze.

R¹ und R² haben dabei die oben angegebenen bevorzugten, besonders bevorzugten, ganz besonders bevorzugten bzw. insbesondere bevorzugten Bedeutungen.

Neu sind Dithiin-diisoimide der Formel (V-a) 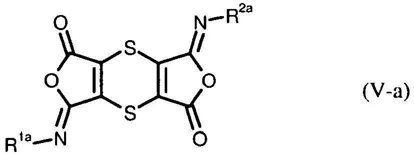 in welcher R^{1a} und R^{2a} die oben angegebenen Bedeutungen haben.

R^{1a} und R^{2a} haben dabei die oben angegebenen bevorzugten, besonders bevorzugten, ganz besonders bevorzugten bzw. insbesondere bevorzugten Bedeutungen.

Beispielsweise wird in einem zweiten Verfahren (vgl. US 3,364,229, Synthetic Commun. 2006, 36, 3591-3597) in einer ersten Stufe Dichlormaleinsäureanhydrid der Formel (VI) mit einem Amin der Formel (III) gegebenenfalls in Gegenwart eines Verdünndungsmittels umgesetzt. Anschließend werden dann die so erhaltenen Maleinsaureimide der Formel (VII) mit einer Schwefelquelle (z.B. Schwefelwasserstoff oder Thioharnstoff) umgesetzt. Die entstandenen Dithiin-tetracarboximide der Formel (I-b) können gegebenenfalls anschließend mit Salpetersäure oxidiert werden. Die Herstellung der Dithiin-tetracarboximide der Formel (I) kann durch das folgende Schema veranschaulicht werden (R steht hierin für R¹ oder R²):

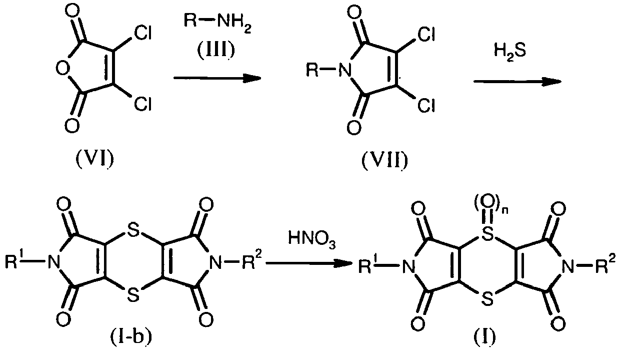

Die vorliegende Erfindung betrifft weiterhin ein Pflanzenschutzmittel zum Bekämpfen unerwünschter Pilze umfassend wenigstens ein Dithiin-tetracarboximid der Formel (I) oder ein Dithiin-diisoimide der Formel (V). Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Außerdem betrifft die Erfindung ein Verfahren zum Bekämpfen unerwünschter Mikroorganismen, dadurch gekennzeichnet, dass man erfindungsgemäß Dithiin-tetracarboximide der Formel (I) oder Dithiin-diisoimide der Formel (V) auf die phytopathogenen Pilze und/oder deren Lebensraum ausbringt.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum. Polyvinylalkohol. Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsfomien, wie Aerosole. Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl-dispergierbare Pulver, Öl-mischbare fließfähige Konzentrate, Öl-mischbare Flüssigkeiten, Schäume, Pasten, Pestizid-ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Fommlierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln. Die Dithiin-tetracarboximide der allgemeinen Formd (I) können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator,
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola: Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; CycloconiumArten, wie beispielsweise Cycloconium oleaginum; Diaporthe-Arten, wie beispielsweise Diaporthe citri: ElsinoeArten, wie beispielsweise Elsinoe fawcettii; Gloeosporium-Arten, wie beispielsweise Gloeosporium laeticolor; Glomerella-Arten, wie beispielsweise Glomerella cingulata: Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum: Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängetkrankheiten, hervorgerufen durch z.B. Corticium-Arten, wie beispielsweise Corticium graminearum; Fusarium-Arten, wie beispielsweise Fusarium oxysporum: Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis ; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea: Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa; Deformationen von Blättern, Blüten und Früchten, hervorgerufen durch z.B. Taphrina-Arten, wie beispielsweise Taphrina deformans:
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea: Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani:
Erkrankungen, hervorgerufen durch bakterielle Erreger wie z.B. Xanthomonas-Arten, wie beispielsweise Xanthomonas campestris pv. oryzae: Pseudomonas-Arten, wie beispielsweise Pseudomonas syringae pv. lachrymans; Erwinia-Arten, wie beispielsweise Erwinia amylovora;

Bevorzugt können die folgenden Krankheiten von Soja-Bohnen bekämpft werden:

Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).
Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Darüber hinaus weisen die erfindungsgemäßen Wirkstoffe auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

### Herstellungsbeispiele

### Herstellung von 2,6-Bis[1-(trifluormethyl)cyclopropyl]-1H,5H-[1,4]dithiino[2,3-c:5,6-c']dipyrrol-1,3,5,7-(2H,6H)-tetron [Verbindung Nr. (7)]

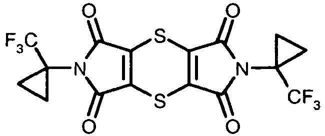

Zu einer Lösung von 0,8 g (3,55 mmol) 4-Oxo-4-{[1-(trifluormethyl)cyclopropyl]amino}butansäure (IV-1) in 2 mL Dioxan wurden unter Eiskühlung (15°C) langsam 7,57 mL (103,75 mmol) Thionylchlorid zugetropft. Man ließ über Nacht auf Raumtemperatur erwärmen und engte die Lösung ein. Der Rückstand wird auf Eis gegossen, mit Essigsäureethylester extrahiert, getrocknet und eingeengt. Nach Chromatographie an Kieselgel (Cyclohexan/Essigsäureethylester 1:1) erhielt man 284 mg (34 % der Theorie) der gewünschten Verbindung.

### Herstellung von (1,3,5,7- Tetraoxo-1,3,5,7-tetrahydro-2H,6H-[1,4]dithiino[2,3-c:5,6-c']dipyrrol-2,6-diyl)dipropan-1,2-diyldiacetat [Verbindung Nr. (36)]

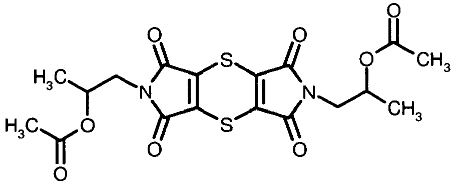

Zu einer Lösung von 1,1 g (3,72 mmol) 1-(3,4-Dichlor-2,5-dioxo-2,5-dihydro-1H-pyrrol-1-yl)propan-2-ylacetat in 10 mL Ethanol gab man 0,283 g (3,72 mmol) an Thioharnstoff und ließ 5 Stunden bei 40°C nachrühren. Nach Abkühlung auf Raumtemperatur saugte man grüne Kristalle ab, die mit Wasser/Ethanol nachgewaschen werden. Das Filtrat wurde mit Wasser und Essigsäureethylester extrahiert, getrocknet und eingeengt. Die Mutterlauge wurde an Kieselgel (Gradient Cyclohexan / Essigsäureethylester 0 % → 100 %) chromatographiert. Man erhielt 0,334 g (39,5 % der Theorie) der gewünschten Verbindung.

### Herstellung von 2,6-Dimethyl-1H,5H-[1,4]dithiino[2,3-c:5,6-c']dipyrrol-1,3,5,7(2H,6H)-tetron-4-oxid [Verbindung Nr. (38)]

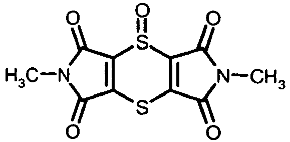

Zu 20 mL eisgekühlter (5°C) rauchender Salpetersäure gab man unter Rühren 3 g (10,63 mmol) 2,6-Dimethyl-1H,5H-[1,4]dithiino[2,3-c:5.6-c']dipyrrol-1,3,5,7(2H,6H)-tetron [Verbindung Nr. (1)]. Nach vollständiger Lösung rührte man 5 min weiter, goss anschließend auf Eiswasser und saugte die gelben Kristalle ab. Man erhielt 2,56 g (80,8 % der Theorie) von der gewünschten Verbindung.

Analog den vorangehenden Beispielen sowie entsprechend den allgemeinen Beschreibungen der Verfahren können die in der folgenden Tabelle 1 genannten Verbindungen der Formel (I) erhalten werden.

**Tabelle 1**

| | | | | |
|---|---|---|---|---|
| 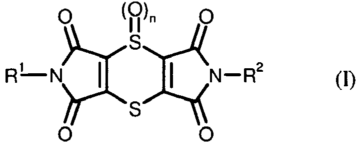 | | | | |

| Nr. | R¹ | R² | n | Physikalische Daten |
|---|---|---|---|---|
| 1 | Me | Me | 0 | Log P = 2,32 |
| 2 | Et | Et | 0 | ¹H-NMR (400 MHz, DMSO-d₆): δ = 1,096; 3,442 ppm |
| 3 | nPr | nPr | 0 | ¹H-NMR (400 MHz, DMSO-d₆): δ = 0,822; 1,566; 3,362 ppm |
| 4 | iPr | iPr | 0 | Log P = 4,19 |
| 5 | cPr | cPr | 0 | ¹H-NMR (400 MHz, DMSO-d₆): δ = 0,50-0,89 ppm |
| 6 | -CH₂CF₃ | -CH₂CF3 | 0 | Log P = 3,41 |
| 7 | 1-(Trifluormethyl)-cyclopropyl | 1-(Trifluormethyl)-cyclopropyl | 0 | Log P = 4,03 |
| 8 | H | H | 0 | Log P = 1,13 |
| 9 | 3,5-Dichlorphenyl | 3,5-Dichlorphenyl | 0 | Fp. > 300°C |
| 10 | Ph | Ph | 0 | Fp. > 300°C |
| 11 | Bz | Bz | 0 | Log P = 4,60 |
| 12 | 2-Methoxyethyl | 2-Methoxyethyl | 0 | Log P = 2,55 |
| 13 | 2-Hydroxybutyl | 2-Hydroxybutyl | 0 | Log P = 2,27 |
| 14 | 2-Hydroxypropyl | 2-Hydroxypropyl | 0 | Log P= 1,63 |
| 15 | 2-Phenoxyethyl | -Phenoxyethyl | 0 | Log P = 3,86 |
| 16 | 2-Ethoxyethyl | 2-Ethoxyethyl | 0 | Log P = 3,24 |
| 17 | 2-Phenylpropan-2-yl | 2-Phenylpropan-2-yl | 0 | ¹H-NMR (400 MHz, DMSO-d₆): δ = 7,20-7,35 ppm |
| 18 | 1-Phenylethyl | 1-Phenylethyl | 0 | ¹H-NMR (400 MHz, DMSO-d₆): δ = 5,197; 5,215; 5,234; 5,251 ppm |
| 19 | 2-Methoxy-2-methylpropyl | 2-Methoxy-2-methylpropyl | 0 | |
| 20 | tBu | tBu | 0 | |
| 21 | -(CH₂)₂OC(=O)CH₃ | 4CH₂)₂OC(=O)CH₃ | 0 | ¹H-NMR (400 MHz, DMSO-d₆): δ = 1,053: 3,654: 4,110 ppm |
| 22 | 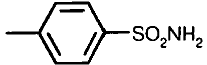 | 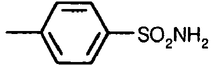 | 0 | ¹H-NMR (400 MHz, DMSO-d₆): δ = 7,492; 7.596: 7,583: 7.946: 7,966 ppm |
| 23 | -CH₂CO₂H | -CH₂CO₂H | 0 | ¹H-NMR (400 MHz, DMSO-d₆): δ = 4,166 ppm |
| 24 | 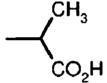 | 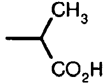 | 0 | Log P = 1,76 |
| 25 | 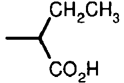 | 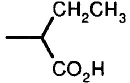 | 0 | |
| 26 | 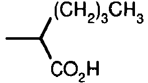 | 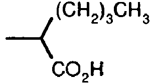 | 0 | |
| 27 | 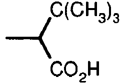 | 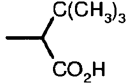 | 0 | ¹H-NMR (400 MHz, DMSO-d₆): δ = 1,620 ppm |
| 28 | 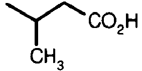 | 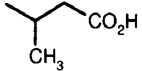 | 0 | Log P = 1,99 |
| 29 | -(CH₂)₄CO₂H | -(CH₂)₄CO₂H | 0 | Log P = 2,02 |
| 30 | 3-(Trifluormethyl)-cyclohexyl | 3-(Trifluormethyl)-cyclohexyl | 0 | ¹³C-NMR (150 MHz, DMSO-d₆): δ = 23,01; 23,71; 27,85: 28,61; 49,19; 126,77; 128,62; 130,56; 164,22 ppm |
| 31 | 3-(Trifluormethyl)-phenyl | 3-(Trifluormethyl)-phenyl | 0 | Log P = 4,91 |
| 32 | 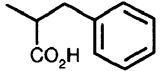 | 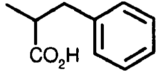 | 0 | Log P = 3,12 |
| 33 | 2-Hydroxyethyl | 2-Hydroxyethyl | 0 | ¹H-NMR (400 MHz, DMSO-d₆): δ = 3,480 ppm |
| 34 | 2-Hydroxy2-methylpropyl | 2-Hydroxy2-methylpropyl | 0 | Log P = 3,65 |
| 35 | 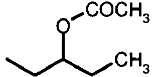 | 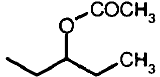 | 0 | Log P = 3,09 |
| 36 | 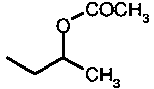 | 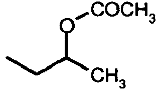 | 0 | Log P = 3,09 |
| 37 | Hydroxymethyl | Hydroxymethyl | 0 | ¹H-NMR (400 MHz, DMSO-d₆): δ = 3,135: 4.789 ppm |
| 38 | Me | Me | 1 | Fp.205°C |
| 39 | H | Et | 0 | Log P = 2,13 |
| 40 | 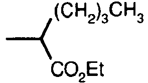 | 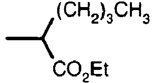 | 0 | Log P = 4,66 |
| 41 | 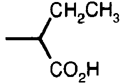 | 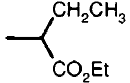 | 0 | Log P = 1,73 |

| | | | | |
|---|---|---|---|---|
| Me = Methyl, Et = Ethyl, nPr = n-Propyl, iPr = Isopropyl, cPr = Cyclopropyl, tBu = tert-Butyl, Bz = Benzyl, Ph = Phenyl | | | | |

### Herstellung von Ausgangstoffen der Formel (IV)

### Herstellung von 4-Oxo-4{[1-(trifluormethyl)cyclopropyl]amino}butansäure (IV-I)

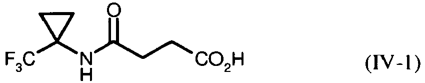

Zu einer Lösung von 496 mg (4,96 mmol) Benrnsteinsäureanhydrid in 10 mL Dioxan gab man unter Eiskühlung (10°C) langsam 800,7 mg (4,96 mmol) 1-(Trifluormethyl)cyclopropanamin und 0,85 mL (4,96 mmol) Diisopropylethylamin zu. Man rührte 20 min. bei Raumtemperatur nach und ließ über Nacht bei dieser Temperatur stehen. Man rührte erneut bei 80°C für 20 min. nach, ließ auf Raumtemperatur abkühlen und engte die Lösung ein. Es wurde mehrfach mit Essigsäureethylester und Wasser im Wechsel gewaschen. Die vereinigten organischen Phasen wurden getrocknet und eingeengt. Man erhielt 815,8 mg (73 % der Theorie) von der gewünschten Verbindung.

Die Bestimmung der in den voranstehenden Tabellen und Herstellungsbeispielen angegebenen Log P-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2,7 mit 0,1 % wässriger Ameisensäure und Acetonitril (enthält 0,1% Ameisensäure) als Eluenten; linearer Gradient von 10% Acetonitril bis 95% Acetonitril

Die Eichung erfolgt mit unverzweigten Alkan-2-onen (mit 3 bis 16 Kohlenstoffatomen), deren logP-Werte bekannt sind (Bestimmung der Log P-Werte anhand der Retentionszeiten durch lineare Interpolation zwischen zwei aufeinanderfolgenden Alkanonen).

### Anwendungsbeispiele

### Beispiel A: Phytophthora-Test (Tomate) / protektiv (nicht erfindungsgemäß)

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßige Wirkstoffzubereitung verwischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Phytophthora infestans* inokuliert. Die Pflanzen werden dann in einer Inkubationskabine bei ca. 20°C und 100% relativer Luftfeuchtigkeit aufgestellt. 3 Tage nach der Inokulation erfolgt die Auswertung. Dabei bedeutet 0 % ein Wirkungsgrad, der demjenigen der Kontrolle entspricht, während ein Wirkungsgrad, von 100 % bedeutet, dass kein Befall beobachtet wird.

In diesem Test zeigten die erfindungsgemäßen Verbindungen 1, 2 und 3 bei einer Konzentration an Wirkstoff von 250 ppm einen Wirkungsgrad von 70 % oder mehr.

### Beispiel B: Plasmopara-Test (Rebe) / protektiv (nicht erfindungsgemäß)

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Plasmopara viticola* inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21°C und ca. 90 % Luftfeuchtigkeit aufgestellt. Die Pflanzen werden dann angefeuchtet und 1 Tag in eine Inkubationskabine gestellt. 6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigten die erfindungsgemäßen Verbindungen 1, 2 und 3 bei einer Konzentration an Wirkstoff von 250 ppm einen Wirkungsgrad von 70 % oder mehr.

### Beispiel C: Venturia-Test (Apfel) / protektiv (nicht erfindungsgemäß)

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia ineaqualis* inokuliert und verbleiben dann 1 Tag bei ca. 20°C und 100 % relativer Luftfeuchtigkeit in einer Inkubationskabine. Die Pflanzen werden dann im Gewächshaus bei ca. 21°C und einer relativen Luftfeuchtigkeit von ca. 90 % aufgestellt. 10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigten die erfindungsgemäßen Verbindungen 1, 2 und 3 bei einer Konzentration an Wirkstoff von 250 ppm einen Wirkungsgrad von 70 % oder mehr.

### Beispiel D: Alternaria-Test (Tomate) / protektiv (nicht erfindungsgemäß)

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Alternaria solani* inokuliert. Die Pflanzen werden dann in einer Inkubationskabine bei ca. 20°C und 100 % relativer Luftfeuchtigkeit aufgestellt. 3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigten die erfindungsgemäßen Verbindungen 1, 2 und 3 bei einer Konzentration an Wirkstoff von 250 ppm einen Wirkungsgrad von 70 % oder mehr.

### Beispiel E: Botrytis-Test (Gurke) /protektiv (nicht erfindungsgemäß)

| | | |
|---|---|---|
| Lösungsmittel: | 49 | Gewichtsteile N, N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von *Botrytis cinerea* inokuliert und stehen dann 48 h bei 100 % relativer Feuchte und 22°C. Anschließend stehen die Pflanzen bei 96 % relativer Luftfeuchtigkeit und einer Temperatur von 14°C. 5-6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigten die erfindungsgemäßen Verbindungen 1, 2 und 3 bei einer Konzentration an Wirkstoff von 500 ppm einen Wirkungsgrad von 70 % oder mehr.

### Beispiel F: Pyrenophora teres -Test (Gerste) / protektiv (nicht erfindungsgemäß)

| | | |
|---|---|---|
| Lösungsmtittel: | 50 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit einer Sporensuspension von *Pyrenophora teres* besprüht. Die Pflanzen verbleiben 48 Stunden bei 20°C und 100 % relativer Luftfeuchtigkeit in einer Inkubationskabine. Die Pflanzen werden in einem Gewächshaus bei einer Temperatur von ca. 20°C und einer relativen Luftfeuchtigkeit von ca. 80 % aufgestellt.

8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigte die erfindungsgemäße Verbindung 1 bei einer Konzentration an Wirkstoff von 1000 ppm einen Wirkungsgrad von 70 % oder mehr.

## Patentansprüche

1. Verwendung von Dithiin-tetracarboximiden der allgemeinen Formel (I) 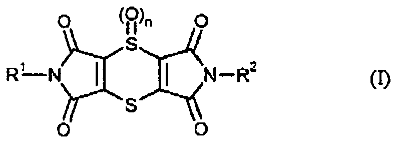 in welcher
R¹ und R² gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
R³ für Wasserstoff, C₁-C₄-Alkyl. C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
R⁴ für Hydroxy, C₁-C₄-Alkyl oder C₁-C₄-Alkoxy steht,
n für 0 oder 1 steht,
zum Schutz von geernteten Früchten oder Gemüse gegen phytopathogene Pilze.

2. Verwendung nach Anspruch 1, **dadurch gekennzeichnet, dass** die Krankheit durch einen der folgenden Erreger hervorgerufen wird: Colletotrichum-Arten, z.B. Colletotrichum gloeosporioides,; Fusarium-Arten, z.B. Fusarium semitectum, Fusarium solani, Fusarium oxysporum; Verticillium-Arten; Botrytis-Arten, z.B. Botrytis cinerea, Phomopsis-Arten; Alternaria-Arten; Phytophthora-Arten, z.B. Phytophthora cactorum; Septoria-Arten; Monilinia-Arten, z.B. Monilinia laxa; Venturia-Arten, z.B. Venturia inaequalis; Glomerella-Arten, z.B. Glomerella cingulata, Sclerotinia-Arten; Penicillium-Arten, z.B. Penicillium expansum; Gloeosporimu-Arten, Stemphyllium-Arten: Thielaviopsis-Arten; Aspergillus-Arten, z.B. Aspergillus niger; Nectria-Arten, z.B. Nectria galligena.

3. Verwendung nach Anspruch 1 oder 2, **dadurch gekennzeichnet, dass** Früchte und Gemüse ausgewählt sind aus Getreide, z.B. Weizen, Gerste, Roggen, Triticale, Reis, Hirse und Hafer; Rüben, z.B. Zuckerrübe und Futterrübe; Kernfrüchte, Steinfrüchte und Beerenfrüchte, z.B. Apfel, Birne, Aprikosen, Kirschen, Mandeln und Pfirsiche wie Erdbeeren; Bohne, Erbse, Soja; Ölpflanzen, z.B. Raps, Senf, Mohn, Olive, Sonnenblume, Kokusnus, Kakao, Erdnuss; Cucurbitaceae, z.B. Gurke; Baumwolle, Zitronen, Orangen und Grapefruit; Brassicaceae, z.B. Weißkohl, Rotkohl, Brokkoli, Blumenkohl, Rosenkohl, Pak Choi, Kohlrabi, Radieschen; Gemüse, z.B. Lauch, Tomate, Zwiebeln, Kartofeln; oder Mais, Tabak, Kaffee, Zuckerrohr, Weinrebe.

4. Verwendung nach Anspruch 1, 2 oder 3, **dadurch gekennzeichnet, dass** die Dithiin-tetracarboximide der allgemeinen Formel (I) ausgewählt sind aus:
(1) 2,6-Dimethyl-1H,5H-[1,4]dithiino[2,3-c:5,6-c']dipyrrol-1,3,5,7(2H,6H)-tetron
(2) 2,6.Diethyl-1H,5H-[1,4]dithiino[2;3-c:5,6-c']dipyrrol-1,3,5,7(2H,6H)-tetron
(3) 2,6-Dipropyl-1H,5H-[1,4]dithiino[2,3-c:5,6-c']dipyrrol-1,3,5,7(2H,6H)-tetron
(4) 2,6-Di(propan-2-yl)-1H,5H-[1,4]dithiino[2,3-c:5,6-c']dipyrrol-1,3,5,7(2H,6H)-tetron
(5) 2,6-Dicyclopropyl-1H,5H-[1,4]dithiino[2,3-c:5,6-c']dipyrrol-1,3,5,7(2H,6H)-tetron
(6) 2,6-Bis(2,2,2-trifluorethyl)-1H,5H-[1,4]dithiino[2,3-c:5,6-c']dipyrrol-1,3,5,7(2H,6H]-tetron
(7) 2,6-Bis[trifluormethyl)cylopropyl]-1H,5H-[1,4]dithiino[2,3-c:5,6-c']dipyrrol-1,3,5,7(2H,6H)-tetron
(8) 1H,5H-[1,4]Dithiino[2,3-c:5,6-c']dipyrrol-1,3,5,7(2H,6H)-tetron
(9) 2,6-Bis(3,5-dichlorphenyl)-1H,5H-[1,4]dithiino[2,3-c:5,6-c']dipyrrol-1,3,5,7(2H,6H)-tetron
(10) 2,6-Diphenyl-1H,5H-[1,4]dithiino[2,3-c:5,6-c']dipyrrol-1,3,5,7(2H,6H)-tetron
(11) 2,6-Dibenzyl-1H,5H-[1,4]dithiino[2,3-c:5,6-c']dipyrrol-1,3,5,7(2H,6H)-tretron
(12) 2,6-Bis(2-methoxyethyl)-1H,5H-[1,4]dithiino[2,3-c:5,6-c']dipyrrol-1,3,5,7(2H,6H)-tetron
(13) 2,6-Bis(2-hydroxybutyl)-1H,5H-[1,4]dithiino[2,3-c:5,6-c']dipyrrol-1,3,5,7(2H,6H)-tetron
(14) 2,6-Bis(2-hydroxypropyl)-1H,5H-[1,4]dithiino[2,3-c:5,6-c']dipyrrol-1,3,5,7(2H,6H)-tetron
(15) 2,6-Bis(2-phenoxyethyl)-1H,5H-[1,4]dithiino[2,3-c:5,6-c']dipyrrol-1,3,5,7(2H,6H)-tetron
(16) 2,6-Bis(2-ethoxyethyl)-1H,5H-[1,4]dithiino[13-c:5,6-c']dipyrrol-1,3,5,7(2H,6H)-tetron
(17) 2,6-Bis(2-phenylpropan-2-yl)-1H,5H-[1,4]dithiino[2,3-c:5,6-c']dipyrrol-1,3,5,7(2H,6H)-tetron
(18) 2,6-Bis(1-phenylethyl)-1H,5H-[1,4]dithiino[2,3-c:5,6-c']dipyrrol-1,3,5,7(2H,6H)-tetron
(19) 2,6-Bis(2-methoxy-2-methylpropyl)-1H,5H-[1,4]dithiino[2,3-c:5,6-c']dipyrrol-1,3,5,7(2H,6H)-tetron
(20) 2,6-Di-tert-butyl-1H,5H-[1,4]dithiino[2,3-c:5,6-c']dipyrrol-1,3,5,7(2H,6H)-tetron
(21) (1,3,5,7-Tetraoxo-1,3,5,7-tetrahydro-2H,6H-[1,4]dithiino[2,3-c:5,6-c']dipyrro-2,6-diyl)diethan-2,1-diyldiacetat
(22) 4,4'-(1,3,5,7-Tetraoxo-1,3,5,7-tetrahydro-2H,6H-[1,4]dithiino[2,3-c:5,6-c']dipyrrol-2,6-diyl)di-benzolsulfonamid
(23) 2,2'-(1,3,5,7-Tetraoxo-1,3,5,7-tetrahydro-2H,6H-[1,4]dithiino[2,3-c:5,6-c']dipyrrol-2,6-diyl)diessigsäure
(24) 2,2'-(1,3,5,7-Tetraoxo-1,3,5,7-tetrahydro-2H,6H-[1,4]dithiino(2,3-c:5,6-c']dipyrrol-2,6-diyl)di-propansäure
(25) 2,2'-(1,3,5,7-Tetraoxo-1,3,5,7-tetrahydro-2H,6H-[1,4]dithiino[2,3-c:5,6-c']dipyrrol-2,6-diyl)dibutan-säure
(26) 2,2'(1,3,5,7-Tetraoxo-1,3,5,7-tetrahydro-2H,6H-[1,4]dithiino[2,3-c:5,6-c']dipyrrol-2,6-diyl)dihexansäure
(27) 2,2'(1,3,5,7-Tetraoxo-1,3,5,7-tetrahydro-2H,6H-[1,4]dithiino[2,3-c:5,6-c']dipyrrol-2,6-diyl)bis(3,3-dimethylbutansäure)
(28) 3,3'-(1,3,5,7-Tetraoxo-1,3,5,7-tetrahydro-2H,6H-[1,4]dithiino[2,3-c:5,6-c']dipyrrol-2,6-diyl)dibutan-säure
(29) 5,5'-(1,3,5,7-Tetraoxo-1,3,5,7-tetrahydro-2H,6H[1,4]dithiino[2,3-c:5,6-c']dipyrrol-2,6-diyl)di-pentansäure
(30) 2,6-Bis[3-(trifluormethyl)cyclohexyl]-1H,5H-[1,4]dithiino[2,3-c:5,6-c']dipyrrol-1,3,5,7(2H,6H)-tetron
(31) 2,6-Bis(3-(trifluormethyl)phenyl]-1H,5H-[1,4]dithiino[2,3-c:5,6-c']dipyrrol-1,3,5,7(2H,6H)-tetron
(32) 2,2'-(1,3,5,7-Tetraoxo-1,3,5,7-tetrahydro-2H,6H-[1,4]dithiino[2,3-c:5,6-c']dipyrrol-2,6-diyl)bis(3-phenylpropansäure)
(33) 2,6-Bis(2-hydroxyethyl)-1H,5H-[1,4]dithiino[2,3-c:5,6-c']dipyrrol-1,3,5,7(2H,6H)-tetron
(34) 2,6-Bis(2-hydroxy-2-methylpropyl)-1H,5H-[1,4]dithiino[2,3-c:5,6-c']dipyrrol-1,3,5,7(2H,6H)-tetron
(35) (1,3,5,7-Tetraoxo-1,3,5,7-tetrahydro-2H,6H-[1,4]dithiino[2,3-c:5,6-c']dipyrrol-2,6-diyl)dibutan-1,2-diyldiacetat
(36) (1,3,5,7-Tetraoxo-1,3,5,7-tetrahydro-2H,6H-[1,4]dithiino[2,3-c:5,6-c']dipyrrol-2,6-diyl)dipropan-1,2-diyldiacetat
(37) 2,6-Bis(hydroxymethyl)-1H,5H-[1,4]dithiino[2,3-c:5,6-c']dipyrrol-1,3,5,7(2H,6H)-tetron
(38) 2,6-Dimethyl-1H,5H-[1,4]dithiino[2,3-c:5,6-c']dipyrrol-1,3,5,7(2H,6H)-tetron-4-oxid
(39) 2-Ethyl-1H,5H-[1,4]dithiino[2,3-c:5,6-c']dipyrrol-1,3,5,7(2H,6H)-tetron
(40) Diethyl-2,2'-(1,3,5,7-tetraoxo-1,3,5,7-tetrahydro-2H,6H-[1,4]dithiino[2,3-c:5,6-c']dipyrrol-2,6-diyl)dihexanoat
(41) 2-[2-(1-Ethoxy-1-oxobutan-2-yl)-1,3,5,7-tetraoxo-2,3,5,7-tetrahydro-1H,6H-[1,4]dithiino[2,3-c:5,6-c']dipyrrol-6-yl]butansäure

5. Verwendung nach Anspruch 1, 2, 3 oder 4, **dadurch gekennzeichnet, dass** Früchte und Gemüse ausgewählt sind Kernfrüchte, Steinfrüchte und Beerenfrüchte, z.B. Apfel, Birne, Aprikosen, Kirschen, Mandeln und Pfirsiche wie Erdbeeren; Zitronen, Orangen und Grapefruit; Weinrebe.

6. Verfahren zum Bekämpfen von Nach-Ernte (Post-Harvest) und/oder Lagerkrankheiten durch Behandlung von Früchten und Gemüse mit wenigstens einem Dithiin-tetracarboximid der allgemeinen Formel (I) 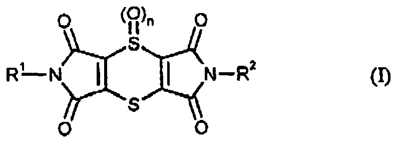 in welcher
R¹ und R² gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-_{C4}-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen.
R³ für Wasserstoff, C₁-C₄-Alkyl C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
R⁴ für Hydroxy, C₁-C₄-Alkyl oder C₁-C₄-Alkoxy steht,
n für 0 oder I steht,
zum Schutz von geernteten Früchten oder Gemüse gegen phytopathogene Pilze.

7. Verfahren nach Anspruch 1, **dadurch gekennzeichnet, dass** die Krankheit durch einen der folgenden Erreger hervorgerufen wird: Colletotrichum-Arten, z.B. Colletotrichum gloeosporioides,; Fusarium-Arten, z.B. Fusarium semitectum, Fusarium solani, Fusarium oxysporum; Verticillium-Arten; Botrytis-Arten, z.B. Botrytis cinerea; Phomopsis-Arten; Alternaria-Arten; Phytophthora-Arten, z.B. Phytophthora cactorum; Septoria-Arten; Monilinia-Arten, z.B. Monilinia laxa; Venturia-Arten, z.B. Venturia inaequalis; Glomerolla-Arten, z.B. Glomerella cingulata; Sclerotinia-Arten; Penicillium-Arten, z.B. Penicillium expansum; Gloeosporium-Arten; Stemphyllium-Arten; Thielaviopsis-Arten; Aspergillus-Arten, z.B. Aspergillus niger; Nectria-Arten, z.B. Nectria galligena.

8. Verfahren nach Anspruch 6 oder 7, **dadurch gekennzeichnet, dass** Früchte und Gemüse ausgewählt sind aus Getreide, z.B. Weizen, Gerste, Roggen, Triticale, Reis, Hirse und Hafer; Rüben, z.B. Zuckerrübe und Futterrübe; Kernfrüchte, Steinfrüchte und Beerenfrüchte, z.B. Apfel, Birne, Aprikosen, Kirschen, Mandeln und Pfirsiche wie Erdbeeren; Bohne, Erbse, Soja; Ölpflanzen, z.B. Raps, Senf, Mohn, Olive, Sonnenblume, Kokusnus, Kakao, Erdnuss; Cucurbitaceae, z.B. Gurke; Baumwolle, Zitronen, Orangen und Grapefruit; Brassicaceae, z.B. Weißkohl, Rotkohl, Brokkoli, Blumenkohl, Rosenkohl, Pak Choi, Kohlrabi, Radieschen; Gemüse, z.B. Lauch, Tomate, Zwiebeln, Kartofeln; oder Mais, Tabak, Kaffee, Zuckerrohr, Weinrebe.

9. Verfahren nach Anspruch 6, 7 oder 8, **dadurch gekennzeichnet, dass** die Dithiin-tetracarboximide der allgemeinen Formel (I) ausgewählt sind aus:
(1) 2,6-Dimethyl-1H,5H-[1,4]dithiino[2,3-c:5,6-c']dipyrrol-1,3,5,7(2H,6H)-tetron
(2) 2,6-Diethyl-1H,5H-[1,4]dithiino[2,3-c:5,6-c']dipyrrol-1,3,5,7(2H,6H)-tetron
(3) 2,6-Dipropyl-1H,5H-[1,4]dithiino[2,3-c:5,6-c']dipyrrol-1,3,5,7(2H,6H)-tetron
(4) 2,6-Di(propan-2-yl)-1H,5H-[1,4]dithiino[2,3-c:5,6-c']dipyrrol-1,3,5,7(2H,6H)-tetron
(5) 2,6-Dicyclopropyl-1H-45-[1,4]dithiino[2,3-c:5,6-c']dipyrrol-1,3,5,7(2H,6H)-tetron
(6) 2,6-Bis(2,2,2-trifluorethyl)-1H,5H-[1,4]dithiino[2,3-c:5,6-c']dipyrrol-1,3,5,7(2H,6H)-tetron
(7) 2,6-Bis[1-(trifluormethyl)cyclopropyl]-1H,5H-[1,4]dithiino[2,3-c:5,6-c']dipyrrol-1,3,5,7(2H,6H)-tetron
(8) 1H,5H-[1,4]Dithiino[2,3-c:5,6-c']dipyrrol-1,3,5,7(2H,6H)-tetron
(9) 2,6-Bis(3,5-dichlorphenyl)-1H,5H-[1,4]dithiino[2,3-c:5,6-c']dipyrrol-1,3,5,7(2H,6H)-tetron
(10) 2,6-Diphenyl-1H,5H-[1,4]dithiino[2,3-c:5,6-c']dipyrrol-1,3,5,7(2H,6H)-tetron
(11) 2,6-Dibenzyl-1H,5H-[1,4]dithiino[2,3-c:5,6-c']dipyrrol-1,3,5,7(2H,6H)-tetron
(12) 2,6-Bis(2-methoxyethyl)-1H,5H-[1,4]dithiino[2,3-c:5,6-c']dipyrrol-1,3,5,7(2H,6H)-tetron
(13) 2,6-Bis(2-hydroxybutyl)-1H,5H-[1,4]dithiino[2,3-c:5,6-c']dipyrrol-1,3,5,7(2H,6H)-tetron
(14) 2,6-Bis(2-hydroxypropyl)-1H,5H-[1,4]dithiino[2,3-c:5,6-c']dipyrrol-1,3,5,7(2H,6H)-tetron
(15) 2,6-Bis(2-phenoxyethyl)-1H,5H-[1,4]dithiino[2,3-c:5,6-c']dipyrrol-1,3,5,7(2H,6H)-tetron
(16) 2,6-Bis(2-ethoxyethyhyl)-1H,5H-[1,4]dithiino[2,3-c:5,6-c']dipyrrol-1,3,5,7(2H,6H)-tetron
(17) 2,6-Bis(2-phenylpropan-2-yl)-1H,5H-[1,4]dithiino[2,3-c:5,6-c']dipyrrol-1,3,5,7(2H,6H)-tetron
(18) 2,6-Bis(1-phenylethyl)-1H,5H-[1,4]dithiino[2,3-c:5,6-c']dipyrrol-1,3,5,7(2H,6H)-tetron
(19) 2,6-Bis(2-methoxy-2-methylpropylpopyl)-1H,5H-[1,4]dithiino[2,3-c:5,6-c']dipyrrol-1,3,5,7(2H,6H)-tetron
(20) 2,6-Di-tert-butyl-1H,5H-[1,4]dithiino[2,3-c:5,6-c']dipyrrol-1,3,5,7(2H,6H)-tetron
(21) (1,3,5,7-Tetraoxo-1,3,5,7-tetrahydro-2H,6H-[1,4]dithiino[2,3-c:5,6-c']dipyrrol-2,6-diyl)diethan-2,1-diyldiacetat
(22) 4,4'-(1,3,5,7-Tetraoxo-1,3,5,7-tetrahydro-2H,6H-[14]dithiino[2,3-c:5,6-c']dipyrrol-2,6-diyl)dibenzolsulfenamid
(23) 2,2-(1,3,5,7-Tetraoxo-1,3,5,7-tetrahydro-2H,6H-[1,4]dithiino[2,3-c:5,b-c']dipyrrol-2,6-diyl)diessigsäure
(24) 2,2'-(1,3,5,7-Tetraoxo-1,3,5,7-tetrahydro-2H,6H-[1,4]dithiino[2,3-c:5,6-c']dipyrrol-2,6-diyl)dipropansäure
(25) 2,2'(1,3,5,7-Tetraoxo-1,3,5,7-tetrahydro-2H,6H-[1,4]dithiino[2,3-c:5,6-c']dipyrrol-2,6-diyl)dibutansäure
(26) 2,2'-(1,3,5,7-Tetraoxo-1,3,5,7-tetrahydro-2H,6H-[1,4]dithiino(2,3-c:5,6-c']dipyrrol-2,6-diyl)dihexansäure
(27) 2.2'-(1,3,5,7-Tetraoxo-1,3,5,7-tetrahydro-2H,6H-[1,4]dithiino(2,3-c:5,6-c']dipyrrol-2,6-diyl)bis(3,3-dimethylbutansäure)
(28) 3,3'-(1,3,5,7-Tetraoxo-1,3,5,7-tetrahydro-2H,6H-[1,4]dithiino[2,3-c:5,6-c']dipyrrol-2,6-diyl)dibutansäure
(29) 5,5'-(1,3,5,7-Tetraoxo-1,3,5,7-tetrahydro-2H,6H-[1,4]dithiino(2,3-c:5,6-c']dipyrrol-2,6-diyl)dipentansäure
(30) 2,6-Bis[3-(trifluormethyl)yclohexyl]-1H,5H-[1,4]dithiino[2,3-c:5,6-c']dipyrrol-1,3,5,7(2H,6H)-tetron
(31) 2,6-Bis[3-(trifluormethyl)phenyl]-1H,5H-[1,4]dithiino[2,3-c:5,6-c']dipyrrol-1,3,5,7(2H,6H)-tetron
(32) 2,2'(1,3,5,7-Tetraoxo-1,3,5,7-tetrahydro-2H,6H-[1,4]dithiino[2,3-c:5,6-c']dipyrrol-2,6-diyl)bis(3-phenylpropansäure)
(33) 2,6-Bis(2-hydroxyethyl)-1H,5H-[1,4]dithiino[2,3-c:5,6-c']dipyrrol-1,3,5,7(2H,6H)-tetron
(34) 2,6-Bis(2-hydroxy-2-methylpropyl)-1H,5H-[1,4]dithiino[2,3-c:5,6-c']dipyrrol-1,3,5,7(2H,6H)-tetron
(35) (1.3,7-Tetraoxo-1,3,5,7-tetrahydro-2H,6H-[1,4]dithiino[2,3-c:5,6-c']dipyrrol-2,6-diyl)dibutan-1,2-diyldiacetat
(36) (1,3,5,7-Tetraoxo-1,3,5,7-tetrahydro-2H,6H-[1,4]dithiino[2,3-c:5,6-c']dipyrrol-2,6-diyl)dipropan-1,2-diyldiacetat
(37) 2,6-Bis(hydroxymethyl)-1H,5H-[1,4]dithiino[2,3-c:5,6-c']dipyrrol-1,3,5,7(2H,6H)-tetron
(38) 2,6-Dimethyl-1H,5H-[1,4]dithiino[2,3-c:5,6-c']dipyrrol-1,3,5,7(2H,6H)-tetron-4-oxid
(39) 2-Ethyl-1H,5H-[1,4]dithiino[2,3-c:5,6-c']dipyrrol-1,3,5,7(2H,6H)-tetron
(40) Diethyl-2,2'-(1,3,5,7-tetraoxo-1,3,5,7-tetrahydro-2H,6H-[1,4]dithiino[2,3-c:5,6-c']dipyrrol-2,6-diyl)dihexanoat
(41) 2-[2-(1-Ethoxy-1-oxobutan-2-yl)-1,3,5,7-tetraoxo-2,3,5,7-tetrahydro-1H,6H-[1,4]dithiino[2,3-c:5,6-c']dipyrrol-6-yl]butansäure

10. Verfahren nach Anspruch 6, 7, 8 oder 9, **dadurch gekennzeichnet, dass** Früchte und Gemüse ausgewählt sind Kernfrüchte, Steinfrüchte und Beerenfrüchte, z.B. Apfel, Birne, Aprikosen, Kirschen, Mandeln und Pfirsiche wie Erdbeeren; Zitronen, Orangen und Grapefruit; Weinrebe.

## Claims

1. Use of dithiine-tetracarboximides of the general formula (I) 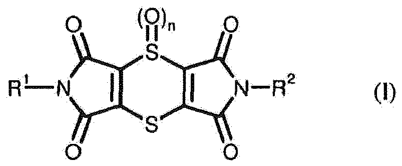 in which
R¹ and R² are identical or different and represent hydrogen, or represent C₁-C₈-alkyl which is optionally monosubstituted or polysubstituted by halogen, -OR³ or -COR⁴, or represent C₃-C₇-cycloalkyl which is optionally monosubstituted or polysubstituted by halogen, C₁-C₄-alkyl or C₁-C₄-haloalkyl, or represent aryl or aryl-(C₁-C₄-alkyl), each of which is optionally monosubstituted or polysubstituted by halogen, C₁-C₄-alkyl, C₁-C₄-haloalkyl, -COR⁴ or sulphonylamino,
R³ represents hydrogen, C₁-C₄-alkyl, C₁-C₄-alkyl-carbonyl, or represents aryl which is optionally monosubstituted or polysubstituted by halogen, C₁-C₄-alkyl or C₁-C₄-haloalkyl,
R⁴ represents hydroxyl, C₁-C₄-alkyl or C₁-C₄-alkoxy,
n represents 0 or 1,
for protecting harvested fruits or vegetables against phytopathogenic fungi.

2. Use according to Claim 1, **characterized in that** the disease is caused by one of the following pathogens: Colletotrichum species, for example Colletotrichum gloeosporioides; Fusarium species, for example Fusarium semitectum, Fusarium solani, Fusarium oxysporum; Verticillium species; Botrytis species, for example Botrytis cinerea; Phomopsis species; Alternaria species; Phytophthora species, for example Phytophthora cactorum; Septoria species; Monilinia species, for example Monilinia laxa; Venturia species, for example Venturia inaequalis; Glomerella species, for example Glomerella cingulata; Sclerotinia species; Penicillium species, for example Penicillium expansum; Gloeosporium species; Stemphyllium species; Thielaviopsis species; Aspergillus species, for example Aspergillus niger; Nectria species, for example Nectria galligena.

3. Use according to Claim 1 or 2, **characterized in that** fruits and vegetables are selected from among cereals, for example wheat, barley, rye, triticale, rice, millet/sorghum and oats; beet, for example sugar beet and fodder beet; pome fruit, stone fruit and soft fruit, for example apple, pear, apricots, cherries, almonds and peaches and also strawberries; bean, pea, soybean; oil crops, for example oilseed rape, mustard, poppy, olive, sunflower, coconut, cacao, peanut; Cucurbitaceae, for example cucumber; cotton, lemons, oranges and grapefruit; Brassicaceae, for example white cabbage, red cabbage, broccoli, cauliflower, Brussels sprouts, pak choi, kohlrabi, radish; vegetables, for example leek, tomato, onions, potatoes; or maize, tobacco, coffee, sugar cane, grape vine.

4. Use according to Claim 1, 2 or 3, **characterized in that** the dithiine-tetracarboximides of the general formula (I) are selected from among:
(1) 2,6-dimethyl-1H,5H-[1,4]dithiino[2,3-c:5,6-c'] dipyrrolo-1,3,5,7(2H,6H)tetrone
(2) 2,6-diethyl-1H,5H-[1,4]dithiino[2,3-c:5,6-c'] dipyrrolo-1,3,5,7(2H,6H)tetrone
(3) 2,6-dipropyl-1H,5H-[1,4]dithiino[2,3-c:5,6-c'] dipyrrolo-1,3,5,7(2H,6H)tetrone
(4) 2,6-di(propan-2-yl)-1H,5H-[1,4]dithiino[2,3-c:5,6-c']dipyrrolo-1,3,5,7(2H,6H)tetrone
(5) 2,6-dicyclopropyl-1H,5H-[1,4]dithiino[2,3-c:5,6-c']dipyrrolo-1,3,5,7(2H,6H)tetrone
(6) 2,6-bis(2,2,2-trifluoroethyl)-1H,5H-[1,4]dithiino[2,3-c:5,6-c']dipyrrolo-1,3,5,7(2H,6H)tetrone
(7) 2,6-bis[1-(trifluoromethyl)cyclopropyl]-1H,5H-[1,4]dithiino[2,3-c:5,6-c']dipyrrolo-1,3,5,7(2H,6H)tetrone
(8) 1H, 5H-[1,4]dithiino[2,3-c:5,6-c']dipyrrolo-1,3,5,7(2H,6H)tetrone
(9) 2,6-bis(3,5-dichlorophenyl)-1H,5H-[1,4]dithiino[2,3-c:5,6-c']dipyrrolo-1,3,5,7(2H,6H)tetrone
(10) 2,6-diphenyl-1H,5H-[1,4]dithiino[2,3-c:5,6-c']-dipyrrolo-1,3,5,7(2H,6H)tetrone
(11) 2,6-dibenzyl-1H,5H-[1,4]dithiino[2,3-c:5,6-c']-dipyrrolo-1,3,5,7(2H,6H)tetrone
(12) 2,6-bis(2-methoxyethyl)-1H,5H-[1,4]dithiino-[2,3-c:5,6-c']dipyrrolo-1,3,5,7(2H,6H)tetrone
(13) 2,6-bis(2-hydroxybutyl)-1H,5H-[1,4]dithiino-[2,3-c:5,6-c']dipyrrolo-1,3,5,7(2H,6H)tetrone
(14) 2,6-bis(2-hydroxypropyl)-2H,5H-[1,4]dithiino-[2,3-c:5,6-c']dipyrrolo-1,3,5,7(2H,6H)tetrone
(15) 2,6-bis(2-phenoxyethyl)-1H,5H-[1,4]dithiino-[2,3-c:5,6-c']dipyrrolo-1,3,5,7(2H,6H)tetrone
(16) 2,6-bis(2-ethoxyethyl)-1H,5H-[1,4]dithiino-[2,3-c:5,6-c']dipyrrolo-1,3,5,7(2H,6H)tetrone
(17) 2,6-bis(2-phenylpropan-2-yl)-1H,5H-[1,4]dithiino-[2,3-c:5,6-c']dipyrrolo-1,3,5,7(2H,6H)tetrone
(18) 2,6-bis(1-phenylethyl)-1H,5H-[1,4]dithiino-[2,3-c:5,6-c']dipyrrolo-1,3,5,7(2H,6H)tetrone
(19) 2,6-bis(2-methoxy-2-methylpropyl)-1H,5H-[1,4]dithiino[2,3-c:5,6-c']dipyrrolo-1,3,5,7(2H,6H)tetrone
(20) 2,6-di-tert-butyl-1H,5H-[1,4]dithiino[2,3-c:5,6-c']dipyrrolo-1,3,5,7(2H,6H)tetrone
(21) (1,3,5,7-tetraoxo-1,3,5,7-tetrahydro-2H,5H-[1,4]dithiino[2,3-c:5,6-c']dipyrrolo-2,6-diyl)diethane-2,1-diyl diacetate
(22) 4,4'-(1,3,5,7-tetraoxo-1,3,5,7-tetrahydro-2H,6H-[1,4]dithiino[2,3-c:5,6-c']dipyrrolo-2,6-diyl)dibenzenesulphonamide
(23) 2,2'-(1,3,5,7-tetraoxo-1,3,5,7-tetrahydro-2H,6H-[1,4]dithiino[2,3-c:5,6-c']dipyrrolo-2,6-diyl)diacetic acid
(24) 2,2'-(1,3,5,7-tetraoxo-1,3,5,7-tetrahydro-2H,6H-[1,4]dithiino[2,3-c:5,6-c']dipyrrolo-2,6-diyl)dipropanoic acid
(25) 2,2'-(1,3,5,7-tetraoxo-1,3,5,7-tetrahydro-2H,6H-[1,4]dithiino[2,3-c:5,6-c']dipyrrolo-2,6-diyl)dibutanoic acid
(26) 2,2'-(1,3,5,7-tetraoxo-1,3,5,7-tetrahydro-2H,6H-[1,4]dithiino[2,3-c:5,6-c']dipyrrolo-2,6-diyl)dihexanoic acid
(27) 2,2'-(1,3,5,7-tetraoxo-1,3,5,7-tetrahydro-2H,6H-[1,4]dithiino[2,3-c:5,6-c']dipyrrolo-2,6-diyl)bis(3,3-dimethylbutanoic acid)
(28) 3,3'-(1,3,5,7-tetraoxo-1,3,5,7-tetrahydro-2H,6H-[1,4]dithiino[2,3-c:5,6-c']dipyrrolo-2,6-diyl)dibutanoic acid
(29) 5,5'-(1,3,5,7-tetraoxo-1,3,5,7-tetrahydro-2H,6H-[1,4]dithiino[2,3-c:5,6-c']dipyrrolo-2,6-diyl)dipentanoic acid
(30) 2,6-bis[3-(trifluoromethyl)cyclohexyl]-1H,5H-[1,4]dithiino[2,3-c:5,6-c']dipyrrolo-1, 3, 5, 7 (2H, 6H) tetrone
(31) 2,6-bis[3-(trifluoromethyl)phenyl]-1H,5H-[1,4]dithiino[2,3-c:5,6-c']dipyrrolo-1,3,5,7(2H,6H)tetrone
(32) 2,2'-(1,3,5,7-tetraoxo-1,3,5,7-tetrahydro-2H,6H-[1,4]dithiino(2,3-c:5,6-c']dipyrrolo-2,6-diyl)bis(3-phenylpropanoic acid)
(33) 2,6-bis(2-hydroxyethyl)-1H,5H-[1,4]-dithiino[2,3-c:5,6-c']dipyrrolo-1,3,5,7(2H,6H)-tetrone
(34) 2,6-bis(2-hydroxy-2-methylpropyl)-1H,5H-[1,4]-dithiino[2,3-c:5,6-c']dipyrrolo-1,3,5,7(2H,6H)-tetrone
(35) (1,3,5,7-tetraoxo-1,3,5,7-tetrahydro-2H,6H-[1,4]dithiino[2,3-c:5,6-c']dipyrrolo-2,6-diyl)dibutane-1,2-diyl diacetate
(36) (1,3,5,7-tetraoxo-1,3,5,7-tetrahydro-2H,6H-[1,4]dithiino[2,3-c:5,6-c']dipyrrolo-2,6-diyl)dipropane-1,2-diyl diacetate
(37) 2,6-bis(hydroxymethyl)-1H,5H-[1,4]-dithiino[2,3-c:5,6-c']dipyrrolo-1,3,5,7(2H,6H)tetrone
(38) 2,6-dimethyl-1H,5H-[1,4]dithiino[2,3-c:5,6-c']-dipyrrolo-1,3,5,7(2H,6H)tetrone 4-oxide
(39) 2-ethyl-1H,5H-[1,4]dithiino[2,3-c:5,6-c']-dipyrrolo-1,3,5,7(2H,6H)tetrone
(40) Diethyl 2,2'-(1,3,5,7-tetraoxo-1,3,5,7-tetrahydro-2H,6H-[1,4]dithiino[2,3-c:5,6-c']dipyrrolo-2,6-diyl)dihexanoate
(41) 2-[2-(1-ethoxy-1-oxobutan-2-yl)-1,3,5,7-tetraoxo-2,3,5,7-tetrahydro-1H,6H-[1,4]dithiino-[2,3-c:5,6-c']dipyrrol-6-yl]butanoic acid.

5. Use according to Claim 1, 2, 3 or 4, **characterized in that** fruits and vegetables are selected from among pome fruit, stone fruit and soft fruit, for example apple, pear, apricots, cherries, almonds and peaches as well as strawberries; lemons, oranges and grapefruit; grape vine.

6. Method for the control of post-harvest diseases and/or storage diseases by treating fruits and vegetables with at least one dithiine-tetracarboximide of the general formula (I) 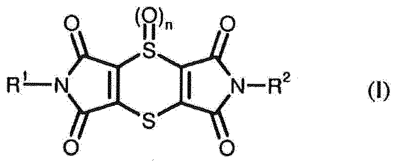 in which
R¹ and R² are identical or different and represent hydrogen, or represent C₁-C₈-alkyl which is optionally monosubstituted or polysubstituted by halogen, -OR³ or -COR⁴, or represent C₃-C₇-cycloalkyl which is optionally monosubstituted or polysubstituted by halogen, C₁-C₄-alkyl or C₁-C₄-haloalkyl, or represent aryl or aryl-(C₁-C₄-alkyl), each of which is optionally monosubstituted or polysubstituted by halogen, C₁-C₄-alkyl, C₁-C₄-haloalkyl, -COR⁴ or sulphonylamino,
R³ represents hydrogen, C₁-C₄-alkyl, C₁-C₄-alkyl-carbonyl, or represents aryl which is optionally monosubstituted or polysubstituted by halogen, C₁-C₄-alkyl or C₁-C₄-haloalkyl,
R⁴ represents hydroxyl, C₁-C₄-alkyl or C₁-C₄-alkoxy,
n represents 0 or 1,
for protecting harvested fruits or vegetables against phytopathogenic fungi.

7. Method according to Claim 6, **characterized in that** the disease is caused by one of the following pathogens: Colletotrichum species, for example Colletotrichum gloeosporioides; Fusarium species, for example Fusarium semitectum, Fusarium solani, Fusarium oxysporum; Verticillium species; Botrytis species, for example Botrytis cinerea; Phomopsis species; Alternaria species; Phytophthora species, for example Phytophthora cactorum; Septoria species; Monilinia species, for example Monilinia laxa; Venturia species, for example Venturia inaequalis; Glomerella species, for example Glomerella cingulata; Sclerotinia species; Penicillium species, for example Penicillium expansum; Gloeosporium species; Stemphyllium species; Thielaviopsis species; Aspergillus species, for example Aspergillus niger; Nectria species, for example Nectria galligena.

8. Method according to Claim 6 or 7, **characterized in that** fruits and vegetables are selected from among cereals, for example wheat, barley, rye, triticale, rice, millet/sorghum and oats; beet, for example sugar beet and fodder beet; pome fruit, stone fruit and soft fruit, for example apple, pear, apricots, cherries, almonds and peaches and also strawberries; bean, pea, soybean; oil crops, for example oilseed rape, mustard, poppy, olive, sunflower, coconut, cacao, peanut; Cucurbitaceae, for example cucumber; cotton, lemons, oranges and grapefruit; Brassicaceae, for example white cabbage, red cabbage, broccoli, cauliflower, Brussels sprouts, pak choi, kohlrabi, radish; vegetables, for example leek, tomato, onions, potatoes; or maize, tobacco, coffee, sugar cane, grape vine.

9. Method according to Claim 6, 7 or 8, **characterized in that** the dithiine-tetracarboximides of the general formula (I) are selected from among:
(1) 2,6-dimethyl-1H,5H-[1,4]dithiino[2,3-c:5,6-c'] dipyrrolo-1,3,5,7(2H,6H)tetrone
(2) 2,6-diethyl-1H,5H-[1,4]dithiino[2,3-c:5,6-c'] dipyrrolo-1,3,5,7(2H,6H)tetrone
(3) 2,6-dipropyl-1H,5H-[1,4]dithiino[2,3-c:5,6-c'] dipyrrolo-1,3,5,7(2H,6H)tetrone
(4) 2,6-di(propan-2-yl)-1H,5H-[1,4]dithiino[2,3-c:5,6-c']dipyrrolo-1,3,5,7(2H,6H)tetrone
(5) 2,6-dicyclopropyl-1H,5H-[1,4]dithiino[2,3-c:5,6-c']dipyrrolo-1,3,5,7(2H,6H)tetrone
(6) 2,6-bis(2,2,2-trifluoroethyl)-1H,5H-[1,4]dithiino[2,3-c:5,6-c']dipyrrolo-1,3,5,7(2H,6H)tetrone
(7) 2,6-bis[1-(trifluoromethyl)cyclopropyl]-1H,5H-[1,4]dithiino[2,3-c:5,6-c']dipyrrolo-1,3,5,7(2H,6H)tetrone
(8) 1H,5H-[1,4]dithiino[2,3-c:5,6-c']dipyrrolo-1,3,5,7(2H,6H)tetrone
(9) 2,6-bis(3,5-dichlorophenyl)-1H,5H-[1,4]dithiino[2,3-c:5,6-c']dipyrrolo-1,3,5,7(2H,6H)tetrone
(10) 2,6-diphenyl-1H,5H-[1,4]dithiino[2,3-c:5,6-c']-dipyrrolo-1,3,5,7(2H,6H)tetrone
(11) 2,6-dibenzyl-1H,5H-[1,4]dithiino[2,3-c:5,6-c']-dipyrrolo-1,3,5,7(2H,6H)tetrone
(12) 2,6-bis(2-methoxyethyl)-1H,5H-[1,4]dithiino-[2,3-c:5,6-c']dipyrrolo-1,3,5,7(2H,6H)tetrone
(13) 2,6-bis(2-hydroxybutyl)-1H,5H-[1,4]dithiino-[2,3-c:5,6-c']dipyrrolo-1,3,5,7(2H,6H)tetrone
(14) 2,6-bis(2-hydroxypropyl)-1H,5H-[1,4]dithiino-[2,3-c:5,6-c']dipyrrolo-1,3,5,7(2H,6H)tetrone
(15) 2,6-bis(2-phenoxyethyl)-1H,5H-[1,4]dithiino-[2,3-c:5,6-c']dipyrrolo-1,3,5,7(2H,6H)tetrone
(16) 2,6-bis(2-ethoxyethyl)-1H,5H-[1,4]dithiino-[2,3-c:5,6-c']dipyrrolo-1,3,5,7(2H,6H)tetrone
(17) 2,6-bis(2-phenylpropan-2-yl)-1H,5H-[1,4]dithiino-[2,3-c:5,6-c']dipyrrolo-1,3,5,7(2H,6H)tetrone
(18) 2,6-bis(1-phenylethyl)-1H,5H-[1,4]dithiino-[2,3-c:5,6-c']dipyrrolo-1,3,5,7(2H,6H)tetrone
(19) 2,6-bis(2-methoxy-2-methylpropyl)-1H,5H-[1,4]dithiino[2,3-c:5,6-c']dipyrrolo-1,3,5,7(2H,6H)tetrone
(20) 2,6-di-tert-butyl-1H,5H-[1,4]dithino[2,3-c:5,6-c']dipyrrolo-1,3,5,7(2H,6H)tetrone
(21) (1,3,5,7-tetraoxo-1,3,5,7-tetrahydro-2H,6H-[1,4]dithiino[2,3-c:5,6-c']dipyrrolo-2,6-diyl)diethane-2,1-diyl diacetate
(22) 4,4'-(1,3,5,7-tetraoxo-1,3,5,7-tetrahydro-2H,6H-[1,4]dithiino[2,3-c:5,6-c']dipyrrolo-2,6-diyl)dibenzenesulphonamide
(23) 2,2'-(1,3,5,7-tetraoxo-1,3,5,7-tetrahydro-2H,6H-[1,4]dithiino[2,3-c:5,6-c']dipyrrolo-2,6-diyl)diacetic acid
(24) 2,2'-(1,3,5,7-tetraoxo-1,3,5,7-tetrahydro-2H,6H-[1,4]dithiino[2,3-c:5,6-c']dipyrrolo-2,6-diyl)dipropanoic acid
(25) 2,2'-(1,3,5,7-tetraoxo-1,3,5,7-tetrahydro-2H,6H-[1,4]dithiino[2,3-c:5,6-c']dipyrrolo-2,6-diyl)dibutanoic acid
(26) 2,2'-(1,3,5,7-tetraoxo-1,3,5,7-tetrahydro-2H,6H-[1,4]dithiino[2,3-c:5,6-c']dipyrrolo-2,6-diyl)dihexanoic acid
(27) 2,2'-(1,3,5,7-tetraoxo-1,3,5,7-tetrahydro-2H,6H-[1,4]dithiino[2,3-c:5,6-c']dipyrrolo-2,6-diyl)bis(3,3-dimethylbutanoic acid)
(28) 3,3'-(1,3,5,7-tetraoxo-1,3,5,7-tetrahydro-2H,6H-[1,4]dithiino[2,3-c:5,6-c']dipyrrolo-2,6-diyl)dibutanoic acid
(29) 5,5'-(1,3,5,7-tetraoxo-1,3,5,7-tetrahydro-2H,6H-[1,4]dithiino[2,3-c:5,6-c']dipyrrolo-2,6-diyl)dipentanoic acid
(30) 2,6-bis[3-(trifluoromethyl)cyclohexyl]-1H,5H-[1,4]dithiino[2,3-c:5,6-c']dipyrrolo-1,3,5,7(2H,6H)tetrone
(31) 2,6-bis[3-(trifluoromethyl)phenyl]-1H,5H-[1,4]dithiino[2,3-c:5,6-c']dipyrrolo-1,3,5,7(2H,6H)tetrone
(32) 2,2'-(1,3,5,7-tetraoxo-1,3,5,7-tetrahydro-2H,6H-[1,4]dithiino(2,3-c:5,6-c']dipyrrolo-2,6-diyl)bis(3-phenylpropanoic acid)
(33) 2,6-bis(2-hydroxyethyl)-1H,5H-[1,4]-dithiino[2,3-c:5,6-c']dipyrrolo-1,3,5,7(2H,6H)-tetrone
(34) 2,6-bis(2-hydroxy-2-methylpropyl)-1H,5H-[1,4]-dithiino[2,3-c:5,6-c']dipyrrolo-1,3,5,7(2H,6H)-tetrone
(35) (1,3,5,7-tetraoxo-1,3,5,7-tetrahydro-2H,6H-[1,4]dithiino[2,3-c:5,6-c']dipyrrolo-2,6-diyl)dibutane-1,2-diyl diacetate
(36) (1,3,5,7-tetraoxo-1,3,5,7-tetrahydro-2H,6H-[1,4]dithiino[2,3-c:5,6-c']dipyrrolo-2,6-diyl)dipropane-1,2-diyl diacetate
(37) 2,6-bis(hydroxymethyl)-1H,5H-[1,4]-dithiino[2,3-c:5,6-c']dipyrrolo-1,3,5,7(2H,6H)tetrone
(38) 2,6-dimethyl-1H,5H-[1,4]dithiino[2,3-c:5,6-c']-dipyrrolo-1,3,5,7(2H,6H)tetrone 4-oxide
(39) 2-ethyl-1H,5H-[1,4]dithiino[2,3-c:5,6-c']-dipyrrolo-1,3,5,7(2H,6H)tetrone
(40) Diethyl 2,2'-(1,3,5,7-tetraoxo-1,3,5,7-tetrahydro-2H,6H-[1,4]dithiino[2,3-c:5,6-c']dipyrrolo-2,6-diyl)dihexanoate
(41) 2-[2-(1-ethoxy-1-oxobutan-2-yl)-1,3,5,7-tetraoxo-2,3,5,7-tetrahydro-1H,6H-[1,4]dithiino-[2,3-c:5,6-c']dipyrrol-6-yl]butanoic acid.

10. Method according to Claim 6, 7, 8 or 9, **characterized in that** fruits and vegetables are selected from among pome fruit, stone fruit and soft fruit, for example apple, pear, apricots, cherries, almonds and peaches as well as strawberries; lemons, oranges and grapefruit; grape vine.

## Revendications

1. Utilisation de dithiine-tétracarboxamides de formule générale (I) 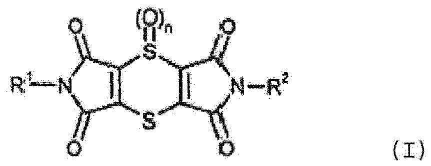 dans laquelle
R¹ et R² sont identiques ou différents et représentent un atome d'hydrogène, un groupe alkyle en C₁-C₈ éventuellement une ou plusieurs fois substitué par halogène, -OR³, -COR⁴, un groupe cycloalkyle en C₃-C₇ éventuellement une ou plusieurs fois substitué par halogène, alkyle en C₁-C₄ ou halogénoalkyle en C₁-C₄, un groupe aryle ou aryl-alkyle(C₁-C₄) chacun éventuellement une ou plusieurs fois substitué par halogène, alkyle en C₁-C₄, halogénoalkyle en C₁-C₄, -COR⁴ ou sulfonylamino,
R³ représente un atome d'hydrogène, un groupe alkyle en C₁-C₄, alkyl(C₁-C₄)carbonyle ou représente un groupe aryle éventuellement une ou plusieurs fois substitué par halogène, alkyle en C₁-C₄ ou halogénoalkyle en C₁-C₄,
R⁴ représente un groupe hydroxy, alkyle en C₁-C₄ ou alcoxy en C₁-C₄,
n représente 0 ou 1,
pour la protection de fruits ou légumes récoltés, contre des champignons phytopathogènes.

2. Utilisation selon la revendication 1, **caractérisée en ce que** la maladie est causée par l'un des agents pathogènes suivants : des espèces de *Colletotrichum,* par exemple *Colletotrichum gloeosporioides ;* des espèces de *Fusarium,* par exemple *Fusa rium semitectum, Fusa rium solani, Fusarium oxysporum ;* des espèces de *Verticillium ;* des espèces de *Botrytis,* par exemple *Botrytis cinerea ;* des espèces de *Phomopsis ;* des espèces *d'Alternaria ;* des espèces de *Phytophthora,* par exemple *Phytophthora cactorum ;* des espèces de *Septoria ;* des espèces de *Monilinia,* par exemple *Monilinia laxa ;* des espèces de *Venturia,* par exemple *Venturia inaequalis ;* des espèces de *Glomerella,* par exemple *Glomerella cingulata ;* des espèces de *Sclerotinia ;* des espèces de *Penicillium,* par exemple *Penicillium expansum ;* des espèces de *Gloeosporzum ;* des espèces de *Stemphyllium ;* des espèces de *Thielaviopsis ;* des espèces *d'Aspergillus,* par exemple *Aspergillus niger ;* des espèces de *Nectria,* par exemple *Nectria galligena.*

3. Utilisation selon la revendication 1 ou 2, **caractérisée en ce que** les fruits et légumes sont choisis parmi les céréales, par exemple le blé, l'orge, le seigle, le triticale, le riz, le millet et l'avoine ; les betteraves, par exemple la betterave sucrière et la betterave fourragère ; les fruits à pépins, les fruits à noyau et les fruits en baies, par exemple la pomme, la poire, l'abricot, les cerises, les amandes et les pêches ainsi que les fraises ; le haricot, le pois, le soja ; les plantes oléagineuses, par exemple le colza, la moutarde, le pavot, l'olive, le tournesol, la noix de coco, le cacao, l'arachide ; les *Cucurbitaceae,* par exemple le concombre ; le coton, les citrons, les oranges et le pamplemousse ; les *Brassicaceae,* par exemple le chou blanc, le chou rouge, le brocoli, le chou-fleur, le pak choi, le chou-rave, les radis ; les légumes, par exemple le poireau, la tomate, les oignons, les pommes de terre ; ou le maïs, le tabac, le café, la canne à sucre, la vigne.

4. Utilisation selon la revendication 1, 2 ou 3, **caractérisée en ce que** les dithiine-tétracarboxamides de formule générale (I) sont choisis parmi les suivants :
(1) 2,6-diméthyl-1H,5H-[1,4]dithiino[2,3-c:5,6-c']-dipyrrol-1,3,5,7(2H,6H)-tétrone
(2) 2,6-diéthyl-1H,5H-[1,4]dithiino[2,3-c:5,6-c']-dipyrrol-1,3,5,7(2H,6H)-tétrone
(3) 2,6-dipropyl-1H,5H-[1,4]dithiino[2,3-c:5,6-c']-dipyrrol-1,3,5,7(2H,6H)-tétrone
(4) 2,6-di(propan-2-yl)-1H,5H-[1,4]dithiino[2,3-c:5,6-c']dipyrrol-1,3,5,7(2H,6H)-tétrone
(5) 2,6-dicyclopropyl-1H,5H-[1,4]dithiino[2,3-c:5,6-c']dipyrrol-1,3,5,7(2H,6H)-tétrone
(6) 2,6-bis(2,2,2-trifluoroéthyl)-1H,5H-[1,4]dithiino-[2,3-c:5,6-c']dipyrrol-1,3,5,7(2H,6H)-tétrone
(7) 2,6-bis[1-(trifluorométhyl)cyclopropyl]-1H,5H-[1,4]dithiino[2,3-c:5,6-c']dipyrrol-1,3,5,7(2H,6H)-têtrone
(8) 1H,5H-[1,4]dithiino[2,3-c:5,6-c']dipyrrol-1,3,5,7(2H,6H)-tétrone
(9) 2,6-bis(3,5-dichlorophényl)-1H,5H-[1,4]dithiino-[2,3-c:5,6-c']dipyrrol-1,3,5,7(2H,6H)-tétrone
(10) 2,6-diphényl-1H,5H-[1,4]dithiino[2,3-c:5,6-c']-dipyrrol-1,3,5,7(2H,6H)-tétrone
(11) 2,6-dibenzyl-1H,5H-[1,4]dithiino[2,3-c:5,6-c']-dipyrrol-1,3,5,7(2H,6H)-tétrone
(12) 2,6-bis(2-méthoxyéthyl)-1H,5H-[1,4]dithiino[2,3-c:5,6-c']dipyrrol-1,3,5,7(2H,6H)-tétrone
(13) 2,6-bis(2-hydroxybutyl)-1H,5H-[1,4]dithiino[2,3-c:5,6-c']dipyrrol-1,3,5,7(2H,6H)-tétrone
(14) 2,6-bis(2-hydroxypropyl)-1H,5H-[1,4]dithiino[2,3-c:5,6-c']dipyrrol-1,3,5,7(2H,6H)-tétrone
(15) 2,6-bis(2-phénoxyéthyl)-1H,5H-[1,4]dithiino[2,3-c:5,6-c']dipyrrol-1,3,5,7(2H,6H)-tétrone
(16) 2,6-bis(2-éthoxyéthyl)-1H,5H-[1,4]dithiino[2,3-c:5,6-c']dipyrrol-1,3,5,7(2H,6H)-tétrone
(17) 2,6-bis(2-phénylpropan-2-y1)-1H,5H-[1,4]dithiino-[2,3-c:5,6-c']dipyrrol-1,3,5,7(2H,6H)-tétrone
(18) 2,6-bis(1-phényléthyl)-1H,5H-[1,4]dithiino[2,3-c:5,6-c']dipyrrol-1,3,5,7(2H,6H)-tétrone
(19) 2,6-bis(2-méthoxy-2-méthylpropyl)-1H,5H-[1,4]-dithiino[2,3-c:5,6-c']dipyrrol-1,3,5,7(2H,6H)-tétrone
(20) 2,6-di-tert-butyl-1H,5H-[1,4]dithiino[2,3-c:5,6-c']dipyrrol-1,3,5,7(2H,6H)-tétrone
(21) diacétate de (1,3,5,7-tétraoxo-1,3,5,7-tétrahydro-2H,6H-[1,4]dithiino[2,3-c:5,6-c']dipyrrol-2,6-diyl)diéthane-2,1-diyle
(22) 4,4'-(1,3,5,7-tétraoxo-1,3,5,7-tétrahydro-2H,6H-[1,4]dithiino[2,3-c:5,6-c']dipyrrol-2,6-diyl)-dibenzènesulfonamide
(23) acide 2,2'-(1,3,5,7-tétraoxo-1,3,5,7-tétrahydro-2H,6H-[1,4]dithiino[2,3-c:5,6-c']dipyrrol-2,6-diyl)diacétique
(24) acide 2,2'-(1,3,5,7-tétraoxo-1,3,5,7-têtrahydro-2H,6H-[1,4]dithiino[2,3-c:5,6-c']dipyrrol-2,6-diyl)dipropionique
(25) acide 2,2'-(1,3,5,7-tétraoxo-1,3,5,7-tétrahydro-2H,6H-[1,4]dithiino[2,3-c:5,6-c']dipyrrol-2,6-diyl)dibutanoïque
(26) acide 2,2'-(1,3,5,7-tétraoxo-1,3,5,7-tétrahydro-2H,6H-[1,4]dithiino[2,3-c:5,6-c']dipyrrol-2,6-diyl)dihexanoïque
(27) acide 2,2'-(1,3,5,7-tétraoxo-1,3,5,7-tétrahydro-2H,6H-[1,4]dithiino[2,3-c:5,6-c']dipyrrol-2,6-diyl)bis(3,3-diméthylbutanoïque)
(28) acide 3,3'-(1,3,5,7-tétraoxo-1,3,5,7-tétrahydro-2H,6H-[1,4]dithiino[2,3-c:5,6-c']dipyrrol-2,6-diyl)dibutanoïque
(29) acide 5,5'-(1,3,5,7-tétraoxo-1,3,5,7-tétrahydro-2H,6H-[1,4]dithiino[2,3-c:5,6-c']dipyrrol-2,6-diyl)dipentanoïque
(30) 2,6-bis[3-(trifluorométhyl)cyclohexyl]-1H,5H-[1,4]dithiino[2,3-c:5,6-c']dipyrrol-1,3,5,7(2H,6H)-tétrone
(31) 2,6-bis[3-(trifluorométhyl)phényl]-1H,5H-[1,4]dithiino[2,3-c:5,6-c']dipyrrol-1,3,5,7(2H,6H)-tétrone
(32) acide 2,2'-(1,3,5,7-tétraoxo-1,3,5,7-tétrahydro-2H,6H-[1,4]dithiino[2,3-c:5,6-c']dipyrrol-2,6-diyl)bis(3-phénylpropionique)
(33) 2,6-bis(2-hydroxyéthyl)-1H,5H-[1,4]dithiino[2,3-c:5,6-c']dipyrrol-1,3,5,7(2H,6H)-tétrone
(34) 2,6-bis(2-hydroxy-2-méthylpropyl)-1H,5H-[1,4]dithiino[2,3-c:5,6-c']dipyrrol-1,3,5,7(2H,6H)-tétrone
(35) diacétate de (1,3,5,7-tétraoxo-1,3,5,7-tétrahydro-2H,6H-(1,4)dithiino[2,3-c:5,6-c']dipyrrol-2,6-diyl)dibutane-1,2-diyle
(36) diacétate de (1,3,5,7-tétraoxo-1,3,5,7-tétrahydro-2H,6H-[1,4]dithiino[2,3-c:5,6-c']dipyrrol-2,6-diyl)dipropane-1,2-diyle
(37) 2,6-bis(hydroxyméthyl)-1H,5H-[1,4]dithiino[2,3-c:5,6-c']dipyrrol-1,3,5,7(2H,6H)-tétrone
(38) 2,6-diméthyl-1H,5H-[1,4]dithiino[2,3-c:5,6-c']-dipyrrol-1,3,5,7(2H,6H)-tétrone-4-oxyde
(39) 2-éthyl-1H,5H-[1,4]dithiino[2,3-c:5,6-c']-dipyrrol-1,3,5,7(2H,6H)-tétrone
(40) 2,2'-(1,3,5,7-tétraoxo-1,3,5,7-tétrahydro-2H,6H-[1,4]dithiino[2,3-c:5,6-c']dipyrrol-2,6-diyl)dihexanoate de diéthyle
(41) acide 2-[2-(1-éthoxy-1-oxobutan-2-yl)-1,3,5,7-tétraoxo-2,3,5,7-tétrahydro-1H,6H-[1,4]dithiino-[2,3-c:5,6-c']dipyrrol-6-yl]butanoïque.

5. Utilisation selon la revendication 1, 2, 3 ou 4, **caractérisée en ce que** les fruits et légumes sont choisis parmi les fruits à pépins, les fruits à noyau et les fruits en baies, par exemple la pomme ; la poire, l'abricot, les cerises, les amandes et les pêches ainsi que les fraises ; les citrons, les oranges et le pamplemousse ; la vigne.

6. Procédé pour la lutte contre des maladies après récole (post-harvest) et/ou au stockage, par traitement des fruits et légumes par au moins un dithiine-tétracarboxamide de formule générale (I) 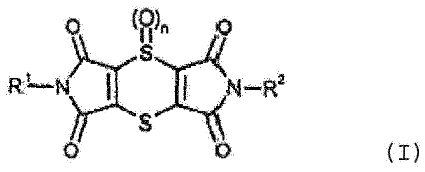 dans laquelle
R¹ et R² sont identiques ou différents et représentent un atome d'hydrogène, un groupe alkyle en C₁-C₈ éventuellement une ou plusieurs fois substitué par halogène, -OR³, -COR⁴, un groupe cycloalkyle en C₃-C₇ éventuellement une ou plusieurs fois substitué par halogène, alkyle en C₁-C₄ ou halogénoalkyle en C₁-C₄, un groupe aryle ou aryl-alkyle(C₁-C₄) chacun éventuellement une ou plusieurs fois substitué par halogène, alkyle en C₁-C₄, halogénoalkyle en C₁-C₄, -COR⁴ ou sulfonylamino,
R³ représente un atome d'hydrogène, un groupe alkyle en C₁-C₄, alkyl(C₁-C₄)carbonyle ou représente un groupe aryle éventuellement une ou plusieurs fois substitué par halogène, alkyle en C₁-C₄ ou halogénoalkyle en C₁-C₄,
R⁴ représente un groupe hydroxy, alkyle en C₁-C₄ ou alcoxy en C₁-C₄,
n représente 0 ou 1,
pour la protection de fruits ou légumes récoltés, contre des champignons phytopathogènes.

7. Procédé selon la revendication 6, **caractérisé en ce que** la maladie est causée par l'un des agents pathogènes suivants : des espèces de *Colletotrichum,* par exemple *Colletotrichum gloeosporioides ;* des espèces de *Fusarium,* par exemple *Fusarium semitectum, Fusarium solani, Fusarium oxysporum ;* des espèces de *Verticillium ;* des espèces de *Botrytis,* par exemple *Botrytis cinerea ;* des espèces de *Phomopsis ;* des espèces *d'Alternaria ;* des espèces de *Phytophthora,* par exemple *Phytophthora cactorum ;* des espèces de *Septoria ;* des espèces de *Monilinia,* par exemple *Monilinia laxa ;* des espèces de *Venturia,* par exemple *Venturia inaequalis ;* des espèces de *Glomerella,* par exemple *Glomerella cingulata ;* des espèces de *Sclerotinia ;* des espèces de *Penicillium,* par exemple *Penicillium expansum ;* des espèces de *Gloeosporium ;* des espèces de *Stemphyllium ;* des espèces de *Thielaviopsis ;* des espèces *d'Aspergillus,* par exemple *Aspergillus niger ;* des espèces de *Nectria,* par exemple *Nectria galligena.*

8. Procédé selon la revendication 6 ou 7, **caractérisé en ce que** les fruits et légumes sont choisis parmi les céréales, par exemple le blé, l'orge, le seigle, le triticale, le riz, le millet et l'avoine ; les betteraves, par exemple la betterave sucrière et la betterave fourragère ; les fruits à pépins, les fruits à noyau et les fruits en baies, par exemple la pomme, la poire, l'abricot, les cerises, les amandes et les pêches ainsi que les fraises ; le haricot, le pois, le soja ; les plantes oléagineuses, par exemple le colza, la moutarde, le pavot, l'olive, le tournesol, la noix de coco, le cacao, l'arachide ; les *Cucurbitaceae,* par exemple le concombre ; le coton, les citrons, les oranges et le pamplemousse ; les *Brassicaceae,* par exemple le chou blanc, le chou rouge, le brocoli, le chou-fleur, le pak choi, le chou-rave, les radis ; les légumes, par exemple le poireau, la tomate, les oignons, les pommes de terre ; ou le maïs, le tabac, le café, la canne à sucre, la vigne.

9. Utilisation selon la revendication 6, 7 ou 8, **caractérisé en ce que** les dithiine-tétracarboxamides de formule générale (I) sont choisis parmi les suivants :
(1) 2,6-diméthyl-1H,5H-[1,4]dithiino[2,3-c:5,6-c']-dipyrrol-1,3,5,7(2H,6H)-tétrone
(2) 2,6-diéthyl-1H,5H-[1,4]dithiino[2,3-c:5,6-c']-dipyrrol-1,3,5,7(2H,6H)-tétrone
(3) 2,6-dipropyl-1H,5H-[1,4]dithiino[2,3-c:5,6-c']-dipyrrol-1,3,5,7(2H,6H)-tétrone
(4) 2,6-di(propan-2-yl)-1H,5H-[1,4]dithiino[2,3-c:5,6-c']dipyrrol-1,3,5,7(2H,6H)-tétrone
(5) 2,6-dicyclopropyl-1H,5H-[1,4]dithiino[2,3-c:5,6-c']dipyrrol-1,3,5,7(2H,6H)-tétrone
(6) 2,6-bis(2,2,2-trifluoroéthyl)-1H,5H-[1,4]dithiino-[2,3-c:5,6-c']dipyrrol-1,3,5,7(2H,6H)-tétrone
(7) 2,6-bis[1-(trifluorométhyl)cyclopropyl]-1H,5H-[1,4]dithiino[2,3-c:5,6-c']dipyrrol-1,3,5,7(2H,6H)-tétrone
(8) 1H,5H-[1,4]dithiino[2,3-c:5,6-c']dipyrrol-1,3,5,7(2H,6H)-tétrone
(9) 2,6-bis(3,5-dichlorophényl)-1H,5H-[1,4]dithiino-[2,3-c:5,6-c']dipyrrol-1,3,5,7(2H,6H)-tétrone
(10) 2,6-diphényl-1H,5H-[1,4]dithiino[2,3-c:5,6-c']-dipyrrol-1,3,5,7(2H,6H)-tétrone
(11) 2,6-dibenzyl-1H,5H-[1,4]dithiino[2,3-c:5,6-c']-dipyrrol-1,3,5,7(2H,6H)-tétrone
(12) 2,6-bis(2-méthoxyéthyl)-1H,5H-[1,4]dithiino[2,3-c:5,6-c']dipyrrol-1,3,5,7(2H,6H)-tétrone
(13) 2,6-bis(2-hydroxybutyl)-1H,5H-[1,4]dithiino[2,3-c:5,6-c']dipyrrol-1,3,5,7(2H,6H)-tétrone
(14) 2,6-bis(2-hydroxypropyl)-1H,5H-[1,4]dithiino[2,3-c:5,6-c']dipyrrol-1,3,5,7(2H,6H)-tétrone
(15) 2,6-bis(2-phénoxyéthyl)-1H,5H-[1,4]dithiino[2,3-c:5,6-c']dipyrrol-1,3,5,7(2H,6H)-tétrone
(16) 2,6-bis(2-éthoxyéthyl)-1H,5H-[1,4]dithiino[2,3-c:5,6-c']dipyrrol-1,3,5,7(2H,6H)-tétrone
(17) 2,6-bis(2-phénylpropan-2-yl)-1H,5H-[1,4]dithiino-[2,3-c:5,6-c']dipyrrol-1,3,5,7(2H,6H)-tétrone
(18) 2,6-bis(1-phényléthyl)-1H,5H-[1,4]dithiino[2,3-c:5,6-c']dipyrrol-1,3,5,7(2H,6H)-tétrone
(19) 2,6-bis(2-méthoxy-2-méthylpropyl)-1H,5H-[1,4]-dithiino[2,3-c:5,6-c']dipyrrol-1,3,5,7(2H,6H)-tétrone
(20) 2,6-di-tert-butyl-1H,5H-[1,4]dithiino[2,3-c:5,6-c']dipyrrol-1,3,5,7(2H,6H)-tétrone
(21) diacétate de (1,3,5,7-tétraoxo-1,3,5,7-têtrahydro-2H,6H-[1,4]dithiino[2,3-c:5,6-c']dipyrrol-2,6-diyl)diéthane-2,1-diyle
(22) 4,4'-(1,3,5,7-tétraoxo-1,3,5,7-tétrahydro-2H,6H-[1,4]dithiino[2,3-c:5,6-c']dipyrrol-2,6-diyl)-dibenzènesulfonamide
(23) acide 2,2'-(1,3,5,7-tétraoxo-1,3,5,7-tétrahydro-2H,6H-[1,4]dithiino[2,3-c:5,6-c']dipyrrol-2,6-diyl)diacétique
(24) acide 2,2'-(1,3,5,7-tétraoxo-1,3,5,7-tétrahydro-2H,6H-[1,4]dithiino[2,3-c:5,6-c']dipyrrol-2,6-diyl)dipropionique
(25) acide 2,2'-(1,3,5,7-tétraoxo-1,3,5,7-tétrahydro-2H,6H-[1,4]dithiino[2,3-c:5,6-c']dipyrrol-2,6-diyl)dibutanoïque
(26) acide 2,2'-(1,3,5,7-tétraoxo-2,3,5,7-tétrahydro-2H,6H-[1,4]dithiino[2,3-c:5,6-c']dipyrrol-2,6-diyl)dihexanoïque
(27) acide 2,2'-(1,3,5,7-tétraoxo-1,3,5,7-tétrahydro-2H,6H-[1,4]dithiino[2,3-c:5,6-c']dipyrrol-2,6-diyl)bis(3,3-diméthylbutanoïque)
(28) acide 3,3'-(1,3,5,7-tétraoxo-1,3,5,7-tétrahydro-2H,6H-[1,4]dithiino[2,3-c:5,6-c']dipyrrol-2,6-diyl)dibutanoïque
(29) acide 5,5'-(1,3,5,7-tétraoxo-1,3,5,7-tétrahydro-2H,6H-[1,4]dithiino[2,3-c:5,6-c']dipyrrol-2,6-diyl)dipentanoïque
(30) 2,6-bis[3-(trifluorométhyl)cyclohexyl]-1H,5H-[1,4]dithiino[2,3-c:5,6-c']dipyrrol-1,3,5,7(2H,6H)-tétrone
(31) 2,6-bis[3-(trifluorométhyl)phényl]-1H,5H-[1,4]dithiino[2,3-c:5,6-c']dipyrrol-1,3,5,7(2H,6H)-tétrone
(32) acide 2,2'-(1,3,5,7-tétraoxo-1,3,5,7-tétrahydro-2H,6H-[1,4]dithiino[2,3-c:5,6-c']dipyrrol-2,6-diyl)bis(3-phénylpropionique)
(33) 2,6-bis(2-hydroxyéthyl)-1H,5H-[1,4]dithiino[2,3-c:5,6-c']dipyrrol-1,3,5,7(2H,6H)-tétrone
(34) 2,6-bis(2-hydroxy-2-méthylpropyl)-1H,5H-[1,4]dithiino[2,3-c:5,6-c']dipyrrol-1,3,5,7(2H,6H)-tétrone
(35) diacétate de (1,3,5,7-tétraoxo-1,3,5,7-tétrahydro-2H,6H-[1,4]dithiino[2,3-c:5,6-c']dipyrrol-2,6-diyl)dibutane-1,2-diyle
(36) diacétate de (1,3,5,7-tétraoxo-1,3,5,7-tétrahydro-2H,6H-[1,4]dithiino[2,3-c:5,6-c']dipyrrol-2,6-diyl)dipropane-1,2-diyle
(37) 2,6-bis(hydroxyméthyl)-1H,5H-[1,4]dithiino[2,3-c:5,6-c']dipyrrol-1,3,5,7(2H,6H)-tétrone
(38) 2,6-diméthyl-1H,5H-[1,4]dithiino[2,3-c:5,6-c']-dipyrrol-1,3,5,7(2H,6H)-tétrone-4-oxyde
(39) 2-éthyl-1H,5H-[1,4]dithiino[2,3-c:5,6-c']-dipyrrol-1,3,5,7(2H,6H)-tétrone
(40) 2,2'-(1,3,5,7-tétraoxo-1,3,5,7-tétrahydro-2H,6H-[1,4]dithiino[2,3-c:5,6-c']dipyrrol-2,6-diyl)dihexanoate de diéthyle
(41) acide 2-[2-(1-éthoxy-1-oxobutan-2-yl)-1,3,5,7-tétraoxo-2,3,5,7-tétrahydro-1H,6H-[1,4]dithiino-[2,3-c:5,6-c']dipyrrol-6-yl]butanoïque.

10. Procédé selon la revendication 6, 7, 8 ou 9, **caractérisée en ce que** les fruits et légumes sont choisis parmi les fruits à pépins, les fruits à noyau et les fruits en baies, par exemple la pomme ; la poire, l'abricot, les cerises, les amandes et les pêches ainsi que les fraises ; les citrons, les oranges et le pamplemousse ; la vigne.
